# EUROPEAN PATENT APPLICATION

(11) **EP 4 831 189 A1**
(43) Date of publication of application: **28.10.2026**
(21) Application number: 24906324.9
(22) Date of filing: 17.12.2024
(51) Int. Cl.: C07K 5/117, C07K 5/103, C07K 5/107, A61K 38/07, A61P 35/00

(54) **KINASE INHIBITOR ACTIVATED IN TUMOR MICROENVIRONMENT, COMPOSITION AND USE**

(30) Priority: 18.12.2023 CN 202311736766
(71) Applicant: Shanghai Affinity Biopharmaceutical Co., Ltd., Shanghai 201203 (CN)
(72) Inventor: LIU, Yuan, Shanghai 201203 (CN); CHEN, Tao, Shanghai 201203 (CN); LIU, Chen, Shanghai 201203 (CN)
(74) Representative: Bjerkén Hynell KB
(86) International application number: PCT/CN2024/139804
(87) International publication number: WO 2025/130843

(57) **Abstract**

A kinase inhibitor activated in a tumor microenvironment, a composition and a use. The kinase inhibitor activated in a tumor microenvironment has a structure shown as R-A-C-D. The present invention also relates to a pharmaceutical composition based on the kinase inhibitor and a use of the kinase inhibitor in preparing a drug for treatment of cancer. The provided kinase inhibitor activated in a tumor microenvironment has good water solubility, the toxic side effects of the kinase inhibitor on normal cells are reduced, and the present invention has great significance for cancer treatment.

## Description

### TECHNICAL FIELD

The present invention relates to the technical field of biomedical synthesis, and specifically relates to a kinase inhibitor activated in a tumor microenvironment, a composition, and a use.

### BACKGROUND

Kinases play an important role in regulating multiple cellular processes, such that kinase inhibitors are developed for use as therapeutic agents in the treatment of a wide range of conditions including cancer. Treating cancer is challenging because it is difficult to kill cancer cells while not affecting normal cells or affecting them to a lower degree. During cancer treatment, killing or otherwise detrimentally affecting normal cells causes side effects in a patient. Therefore, improving drug targeting, reducing toxic side effects of a drug, and avoiding an effect on normal cells are among difficult problems to be addressed in the research and development of drugs for cancer treatment.

A protein kinase inhibitor is an enzyme inhibitor capable of blocking an action of a protein kinase. The protein kinase adds a phosphate group to a protein in a process called phosphorylation, which can turn the protein on or off, thereby affecting an activity and a function level of the protein. Protein kinase inhibitors can penetrate the blood-brain barrier (BBB) for treating brain metastasis and/or leptomeningeal disease; chemotherapy drugs rarely can pass through the blood-brain barrier, and long-term side effects of radiation therapy can also be devastating.

In addition, water solubility is an extremely important physicochemical property of small molecule organic drugs, and is also one of key issues in a process of research and development of small molecule drugs. Good water solubility contributes to exertion of efficacy and improvement of pharmacokinetic properties. In the field of medicinal chemistry, improving water solubility of a drug through a chemical structure modification method is a basic method for considering and solving problems based on the nature of dissolution.

Therefore, in the art, providing a kinase inhibitor having good water solubility and low toxic side effects is of great significance for improving a treatment effect of cancer.

### SUMMARY

To solve the above technical problems, the present invention provides a kinase inhibitor activated in a tumor microenvironment, wherein the kinase inhibitor is a compound having a structure shown as the following formula (I), a stereoisomer thereof, or a pharmaceutically acceptable salt thereof: R-A-C-D (I)
wherein,
R is a modifying group for improving physicochemical properties of a drug;
A is a protease-cleavable amino acid linker;
C is a steric hindrance group or a self-immolative group; and
D is a drug molecule.

Specifically, the R is a modifying group with an MI structure shown as a structure of the following formula (II): 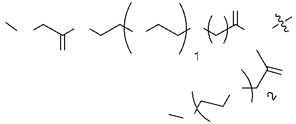 or 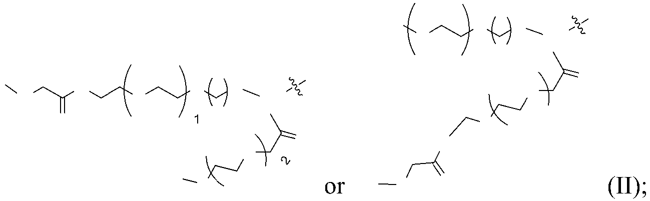 wherein,
MI is a structure having a conjugated system and an addition acceptor;
Rc is a structure for increasing water solubility, compound rigidity, or expanding spatial distance;
n₁ is a positive integer from 1 to 12, that is, any one of n₁ = 1, 2, 3, 4, 5, 6, 7, 8, 9, 10, 11, or 12; n₂ is a positive integer from 1 to 12, that is, any one of n₂ = 1, 2, 3, 4, 5, 6, 7, 8, 9, 10, 11, or 12;
X₁ is a trifunctional group;
and a wavy line represents a connection position of the R and the A.

Specifically, the MI is selected from: 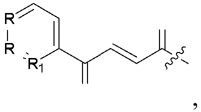 wherein R₁, R₂, and R₃ are CH or N;
or 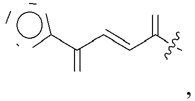 wherein a cyclic structure in the structure is a five-membered ring including any one of N, O, or S, and R₄, R₅, R₆, and R₇ are one of CH, N, O, or S;
or 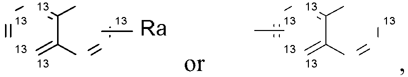 wherein R₈, R₉, R₁₀, R₁₁, R₁₂, R₁₃, R₁₄, and R₁₅ are C, CH, or N, and Ra and Rb are 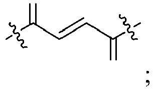 or 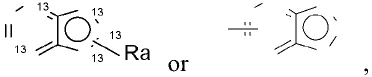 wherein a cyclic structure in the structure is a five-membered ring including any one of N, O, or S, and R₁₆, R₁₇, and R₁₈ are one of C, CH, N, O, or S, R₁₉, R₂₀, R₂₁, and R₂₂ are C, CH, or N, and Re and Rf are

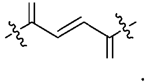

Specifically, the Rc is selected from C₁-₁₂ alkyl, C₁₋₁₂ oxyalkyl-C₁₋₁₂ alkyl, C₁₋₁₂ alkyl-C₃₋₈ cycloalkyl, (C₁₋₄ alkyl-O)p-C₁₋₁₂ alkyl, phenyl-C₁₋₁₂ alkyl, C₃₋₈ cycloalkyl, C₁₋₁₂ alkyl-C₃₋₈ cycloalkyl-C₁₋₁₂ alkyl, C₁₋₁₂ alkyl-phenyl-C₁₋₁₂ alkyl, or C₁₋₁₂ alkylcarbonylamino-(C₁₋₄ alkyl-O)q-C₁₋₁₂ alkyl;
wherein, p is a positive integer from 1 to 12, that is, any one of p = 1, 2, 3, 4, 5, 6, 7, 8, 9, 10, 11, or 12, q is a positive integer from 1 to 12, that is, any one of q = 1, 2, 3, 4, 5, 6, 7, 8, 9, 10, 11, or 12, the phenyl is one or two substituted phenyl groups, and a substituent in the substituted phenyl is a halogen atom.

Specifically, the X₁ is selected from lysine, Lys, or glutamic acid, Glu;
when the X₁ is the lysine, a structure of the X₁ is 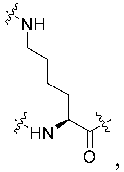 wherein a carboxyl terminus of the X₁ is connected to the A, and two amino termini of the X₁ are connected in a one-to-one correspondence to a side chain containing n1 or a side chain containing n2, that is, the side chain containing n1 can be connected to an amino group on a lysine main chain or can be connected to an amino group on a lysine side chain; and the side chain containing n2 can be connected to the amino group on the lysine main chain or can be connected to the amino group on the lysine side chain;
when the X₁ is the glutamic acid, Glu, a structure of the X₁ is 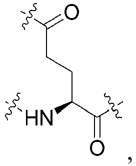 wherein an amino terminus is connected to the side chain containing n1 or the side chain containing n2; a carboxyl terminus adjacent to an amino group on the glutamic acid is connected to the A, and a carboxyl terminus remote from the amino group on the glutamic acid is connected to the side chain containing n1 or the side chain containing n2.

Specifically, the A is -Ala-Ala-Asn-, wherein an Ala terminus is connected to the lysine side chain, and an Asn terminus is connected to the C.

Specifically, the C is 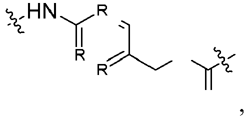 wherein R₂₃, R₂₄, and R₂₅ are CH or N, an amino terminus of the C is connected to the Asn, and a carbonyl terminus of the C is connected to the D.

Specifically, the drug molecule D is one of the following chemical formulas:

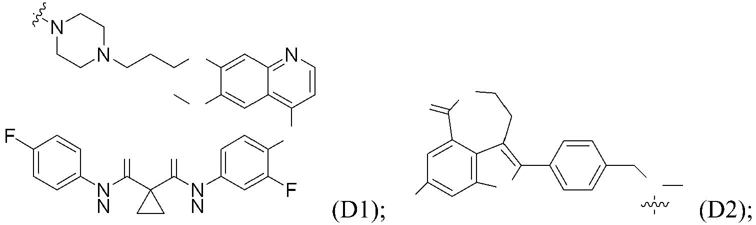

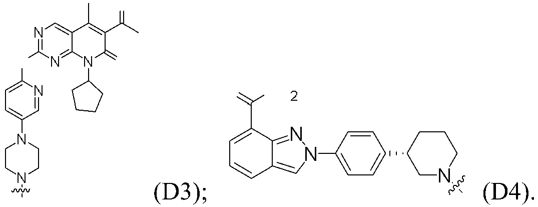

The present invention further provides a pharmaceutical composition, comprising the above kinase inhibitor activated in a tumor microenvironment and a pharmaceutically acceptable carrier thereof, or a pharmaceutical composition.

Therein, the pharmaceutically acceptable carrier is usually safe and non-toxic, and can broadly include any known substance used in the pharmaceutical industry for preparing a pharmaceutical composition, such as a filler, a diluent, a coagulant, a binder, a lubricant, a glidant, a stabilizer, a colorant, a wetting agent, a disintegrant, and the like. Suitable pharmaceutically acceptable carriers include sugars, such as lactose or sucrose, mannitol or sorbitol; cellulose preparations and/or calcium phosphates, for example, tricalcium phosphate or dicalcium phosphate; starch pastes, including corn starch, wheat starch, rice starch, potato starch, gelatin, tragacanth, methylcellulose, hydroxypropyl methylcellulose, sodium carboxymethyl cellulose, and/or polyvinylpyrrolidone; silica, talc, stearic acid or a salt thereof, such as magnesium stearate or calcium stearate, and/or polyethylene glycol, etc. When selecting a pharmaceutically acceptable carrier, a mode of administration of the pharmaceutical composition mainly needs to be considered.

The pharmaceutical composition provided in the present invention contains a therapeutically effective amount of a compound of formula (I) or a pharmaceutically acceptable salt thereof.

A dosage form of the pharmaceutical composition includes, but is not limited to, a tablet, a capsule, or an injection. A route of administration of the pharmaceutical composition includes, but is not limited to, parenteral, subcutaneous, intravenous, intramuscular, intraperitoneal, transdermal, buccal, intrathecal, intracranial, nasal, or topical routes of administration. An administration dose of a drug can be determined according to the age, the health, and the body weight of a patient, a type of concurrent treatment, a frequency of treatment, and the like.

A subject for administering the pharmaceutical composition is a mammal, preferably a human.

The compound of formula (I) or the pharmaceutically acceptable salt thereof or the pharmaceutical composition in the present disclosure is particularly useful for treating cancer or cancer cell metastasis. The cancer that can be treated by the compound of formula (I) or the pharmaceutically acceptable salt thereof or the pharmaceutical composition in the present disclosure can be determined according to therapeutic activity possessed by the drug molecule D of interest per se.

The present invention further provides a use of the aforementioned kinase inhibitor activated in a tumor microenvironment in the preparation of a medicament for treating cancer, the cancer including colorectal cancer, ovarian cancer, fallopian tube cancer, peritoneal cancer, breast cancer, gastric cancer, liver cancer, head and neck cancer, prostate cancer, and pancreatic cancer.

In certain aspects, the compound of formula (I) or the pharmaceutically acceptable salt thereof or the pharmaceutical composition provided in the present invention can further stimulate proliferation and invasion into a lesion of T cells, inhibit tumor-associated macrophages, and/or promote and stimulate an immune response.

Therefore, the present invention further provides a method for treating cancer or cancer cell metastasis, the method comprising administering a therapeutically effective amount of the compound of formula (I) or the pharmaceutically acceptable salt thereof or the pharmaceutical composition disclosed herein to a subject in need thereof. The cancer or the cancer cell metastasis is as described above.

In certain aspects, the present invention further provides a method for stimulating proliferation and invasion into a lesion of T cells, inhibiting tumor-associated macrophages, and/or promoting and stimulating an immune response, the method comprising administering a therapeutically effective amount of the compound of formula (I) or the pharmaceutically acceptable salt thereof or the pharmaceutical composition disclosed herein to a subject in need thereof.

The subject is a mammal, preferably a human.

The present invention further provides a use of the compound of formula (I) or the pharmaceutically acceptable salt thereof in the preparation of a medicament for treating cancer or cancer cell metastasis, or a use in the preparation of an immunotherapy drug. The cancer or the cancer cell metastasis is as described above; the immunotherapy drug can be used for stimulating proliferation and invasion into a lesion of T cells, inhibiting tumor-associated macrophages, and/or promoting and stimulating an immune response.

The present invention further provides the compound of formula (I) or the pharmaceutically acceptable salt thereof for use in treating cancer or cancer cell metastasis, or for use in stimulating proliferation and invasion into a lesion of T cells, inhibiting tumor-associated macrophages, and/or promoting and stimulating an immune response.

The present invention further provides a use for administering the aforementioned compound of formula (I) or the pharmaceutically acceptable salt thereof or the pharmaceutical composition, comprising a use of the aforementioned kinase inhibitor activated in a tumor microenvironment or a pharmaceutically acceptable salt thereof, or the aforementioned composition or a pharmaceutically acceptable salt thereof in combination with an anti-PD-1 antibody in the preparation of a medicament for combined treatment of a tumor.

The present invention further provides a method for treating or preventing cancer, or inhibiting cancer cell proliferation, comprising: administering the aforementioned compound of formula (I) or the pharmaceutically acceptable salt thereof or the pharmaceutical composition to a subject.

Specifically, the method further comprises: administering an immune checkpoint inhibitor to the subject in combination with the aforementioned compound of formula (I) or the pharmaceutically acceptable salt thereof or the pharmaceutical composition.

Specifically, the immune checkpoint inhibitor is an anti-PD-1 antibody drug.

Compared with the prior art, advantageous effects of the present invention include, but are not limited to:
1. For the tumor-targetedly activated kinase inhibitor provided in the present invention, through structural modification of the R group, water solubility of the kinase inhibitor and overall targeting of the kinase inhibitor to a tumor are improved. Through modification of the R group, after the kinase inhibitor provided in the present invention enters a body, the drug molecule D is released and directly enters an interior of the tumor, which improves targeting of the drug to the tumor and reduces toxic side effects of the drug on normal cells of a human body;
2. The tumor microenvironment-activated kinase inhibitor and the pharmaceutical composition thereof provided in the present invention have been verified to be capable of being cleaved by Legumain enzyme to release a drug molecule. Compared with clinical drugs such as foretinib and the like, not only higher targeting to the tumor is achieved, but also a killing effect on cancer cells is significantly improved, and good anti-tumor efficacy is achieved. Meanwhile, the water solubility of the kinase inhibitor is significantly improved, and the toxic side effects of the drug are reduced.

### BRIEF DESCRIPTION OF THE DRAWINGS

FIG. 1 is a chromatogram of Legumain enzymatic cleavage of compound S1 in embodiment 5;
FIG. 2 is a data plot of survival rate of B16F10 cells in an environment of foretinib in embodiment 9;
FIG. 3 is a data plot of survival rate of B16F10 cells in an environment of compound D1 in embodiment 9;
FIG. 4 is a data plot of survival rate of B16F10 cells in an environment of compound S1 in embodiment 9;
FIG. 5 is a data plot of survival rate of A549 cells in an environment of foretinib in embodiment 10;
FIG. 6 is a data plot of survival rate of A549 cells in an environment of compound D1 in embodiment 10;
FIG. 7 is a data plot of survival rate of A549 cells in an environment of compound S1 in embodiment 10;
FIG. 8 is a result plot of the effect of the test substance on growth of mouse xenograft CT-26 in embodiment 11;
FIG. 9 is a result plot of the effect of the test substance on growth of mouse xenograft MKN45 in embodiment 12;
FIG. 10 is a result plot of the effect of the test substance on growth of mouse xenograft HUH7 in embodiment 13;
FIG. 11 is a result plot of tumor volume of mouse colorectal cancer CT-26 model in embodiment 16;
FIG. 12 is a result plot of tumor volume of mouse colorectal cancer CT-26 model in embodiment 17;
FIG. 13 is a result plot of tumor volume of human gastric cancer MKN45 model in embodiment 18.

### DETAILED DESCRIPTION OF THE DISCLOSURE

The technical solutions in the embodiments of the present invention will be clearly and completely described below. Clearly, the described embodiments are only a part of the embodiments of the present invention, rather than all of the embodiments. Based on the embodiments in the present invention, all other embodiments obtained by those of ordinary skill in the art without creative labor shall fall within the protection scope of the present invention. Materials, instruments, reagents, and the like used in the following embodiments can be obtained from commercial sources unless otherwise specified. Technical means used in the embodiments are conventional means well known to those skilled in the art unless otherwise specified.

The kinase inhibitor activated in a tumor microenvironment provided in the present invention is a compound having a structure shown as the following formula (I), a stereoisomer thereof, or a pharmaceutically acceptable salt thereof: R-A-C-D (I)
wherein,
R is a modifying group for improving physicochemical properties of a drug;
A is a protease-cleavable amino acid linker;
C is a steric hindrance group or a self-immolative group; and
D is a drug molecule.

Specifically, the R is a modifying group with an MI structure shown as a structure of the following formula (II): 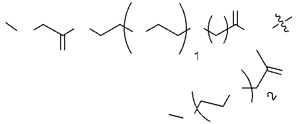 or 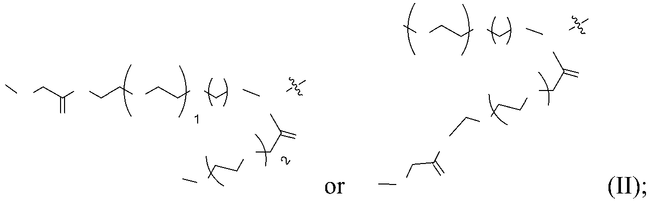 wherein,
MI is a structure having a conjugated system and an addition acceptor;
Rc is a structure for increasing water solubility, compound rigidity, or expanding spatial distance;
n₁ is a positive integer from 1 to 12, that is, any one of n₁ = 1, 2, 3, 4, 5, 6, 7, 8, 9, 10, 11, or 12; n₂ is a positive integer from 1 to 12, that is, any one of n₂ = 1, 2, 3, 4, 5, 6, 7, 8, 9, 10, 11, or 12;
X₁ is a trifunctional group;
and a wavy line represents a connection position of the R and the A.

Specifically, the MI is selected from: 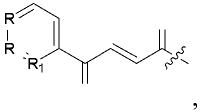 wherein R₁, R₂, and R₃ are CH or N;
or 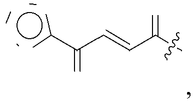 wherein a cyclic structure in the structure is a five-membered ring including any one of N, O, or S, and R₄, R₅, R₆, and R₇ are one of CH, N, O, or S;
or 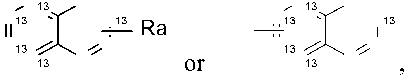 wherein R₈, R₉, R₁₀, R₁₁, R₁₂, R₁₃, R₁₄, and R₁₅ are C, CH, or N, and Ra and Rb are 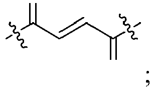 or 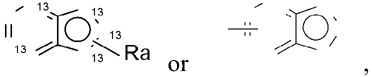 wherein a cyclic structure in the structure is a five-membered ring including any one of N, O, or S, and R₁₆, R₁₇, and R₁₈ are one of C, CH, N, O, or S, R₁₉, R₂₀, R₂₁, and R₂₂ are C, CH, or N, and Re and Rf are

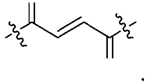

Preferably, the MI structure includes, but is not limited to, the following structures:
selected from

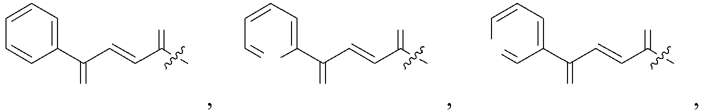

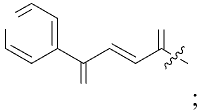

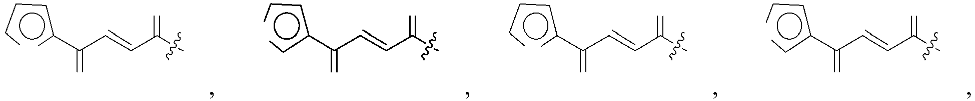

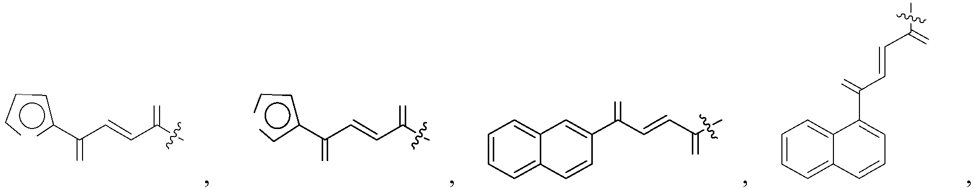

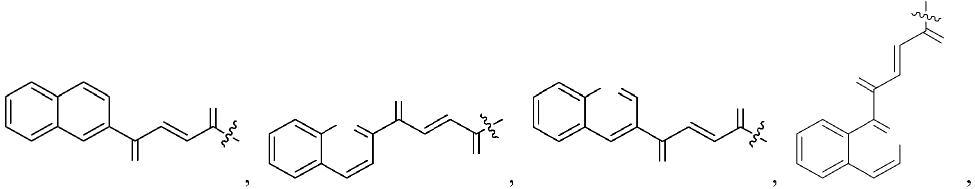

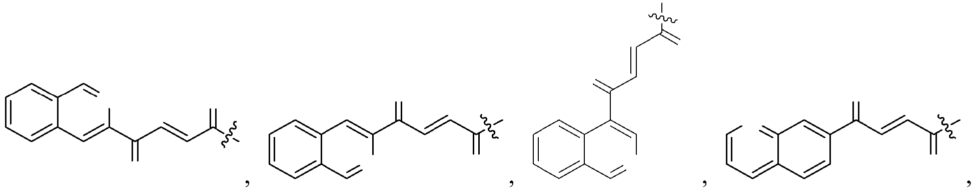

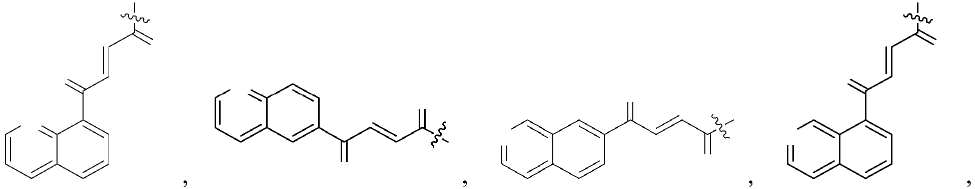

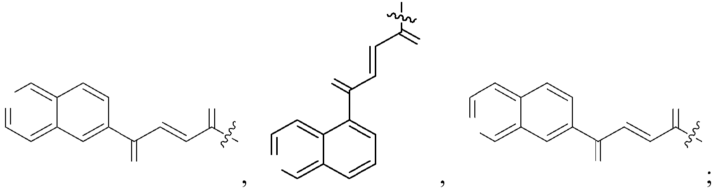

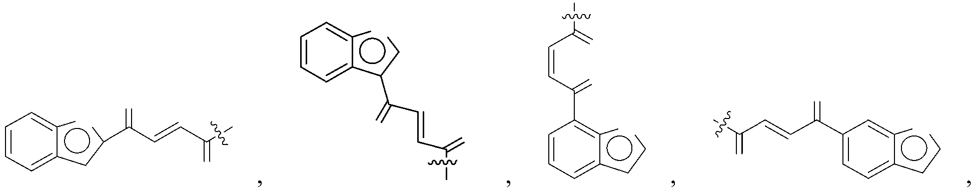

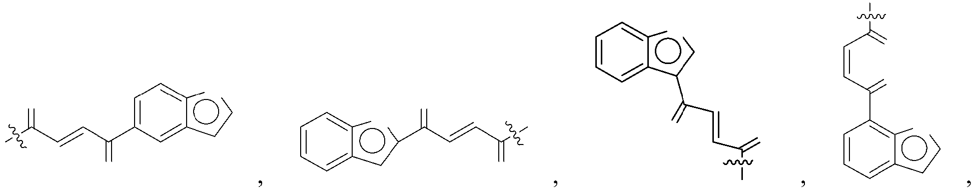

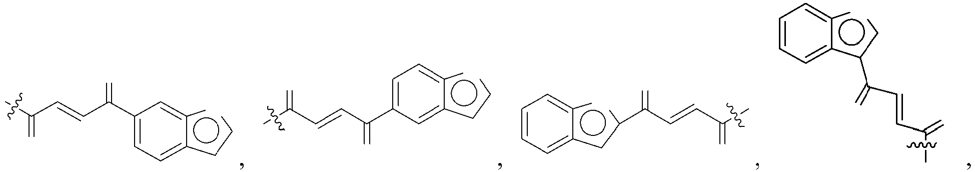

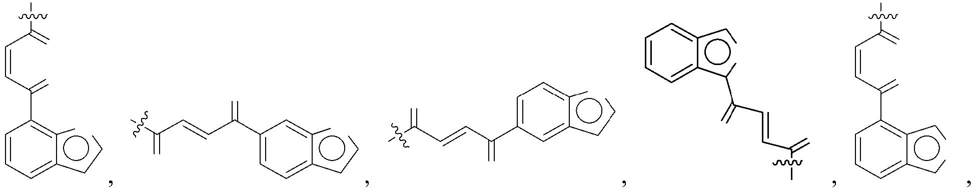

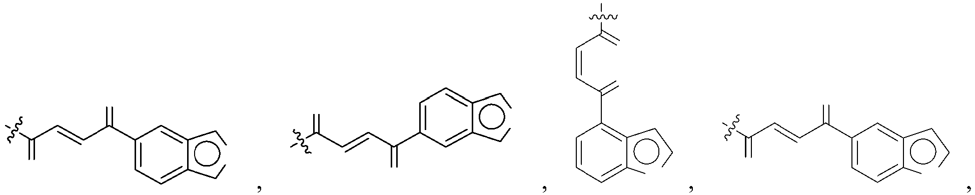

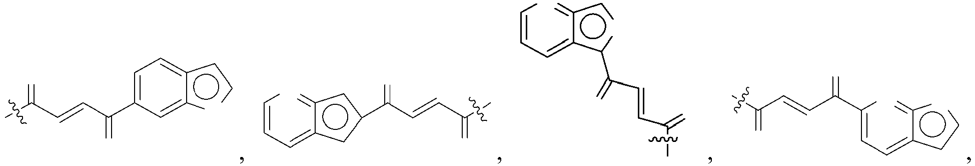

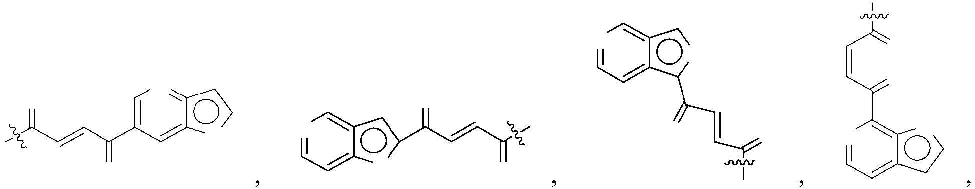

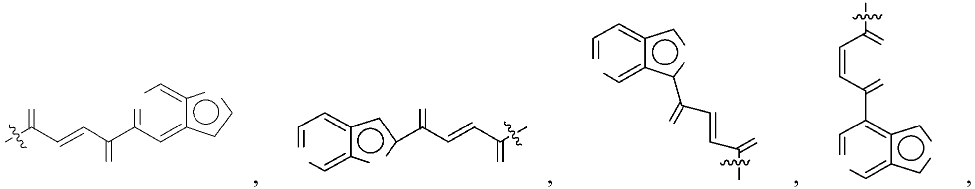

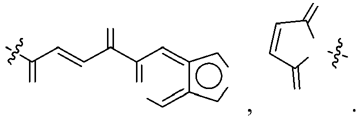

Specifically, the Rc is selected from C₁-₁₂ alkyl, C₁₋₁₂ oxyalkyl-C₁₋₁₂ alkyl, C₁₋₁₂ alkyl-C₃₋₈ cycloalkyl, (C₁₋₄ alkyl-O)p-C₁₋₁₂ alkyl, phenyl-C₁₋₁₂ alkyl, C₃₋₈ cycloalkyl, C₁₋₁₂ alkyl-C₃₋₈ cycloalkyl-C₁₋₁₂ alkyl, C₁₋₁₂ alkyl-phenyl-C₁₋₁₂ alkyl, or C₁₋₁₂ alkylcarbonylamino-(C₁₋₄ alkyl-O)q-C₁₋₁₂ alkyl;
wherein, p is a positive integer from 1 to 12, that is, any one of p = 1, 2, 3, 4, 5, 6, 7, 8, 9, 10, 11, or 12, q is a positive integer from 1 to 12, that is, any one of q = 1, 2, 3, 4, 5, 6, 7, 8, 9, 10, 11, or 12, the phenyl is one or two substituted phenyl groups, and a substituent in the substituted phenyl is a halogen atom.

Preferably, Rc includes, but is not limited to, the following structures:

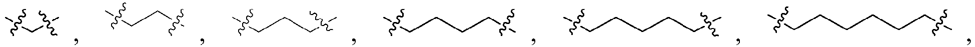

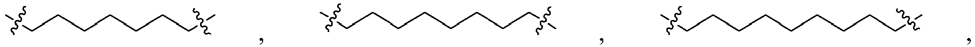

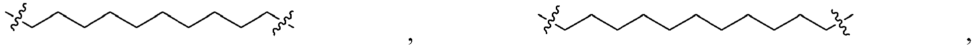

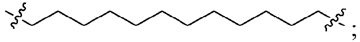

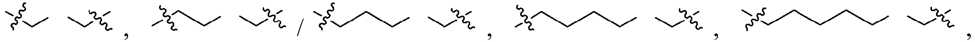

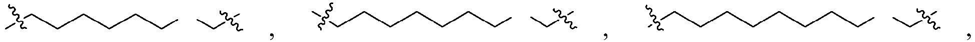

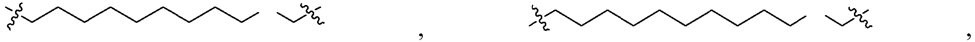

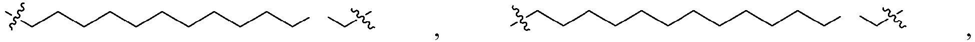

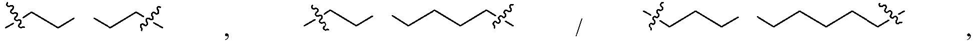

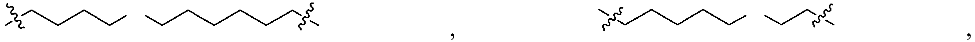

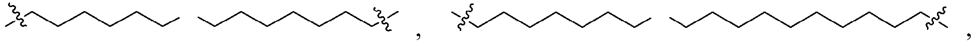

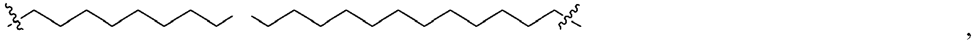

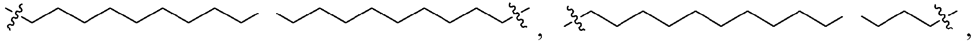

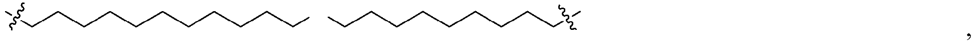

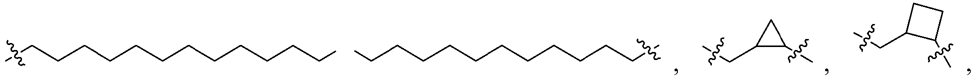

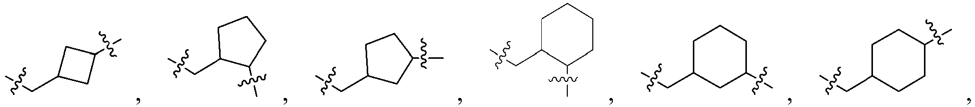

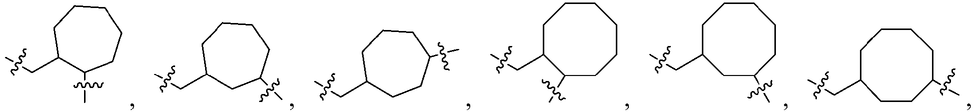

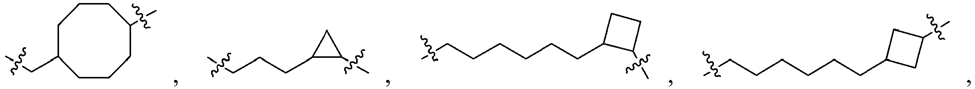

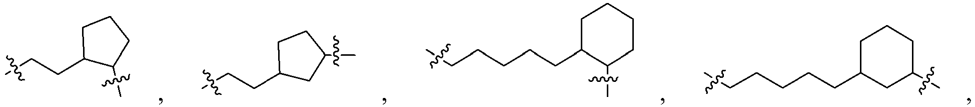

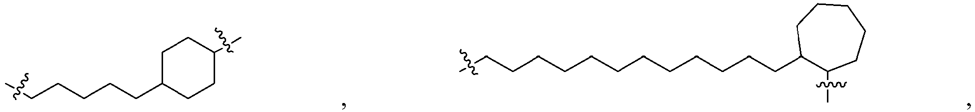

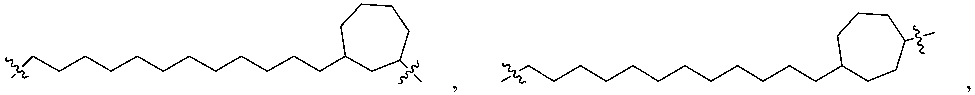

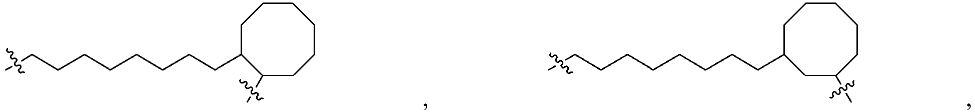

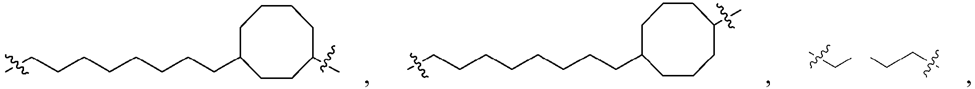

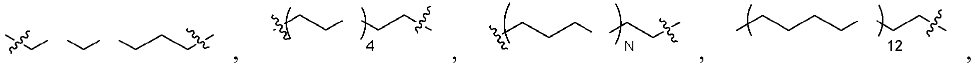

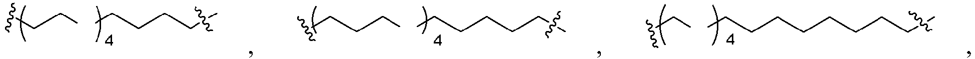

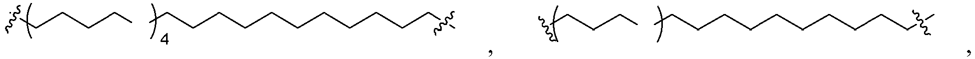

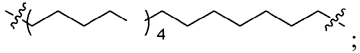

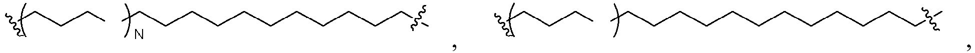

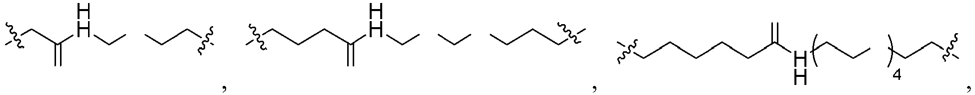

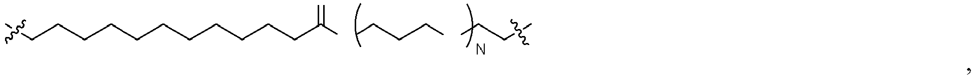

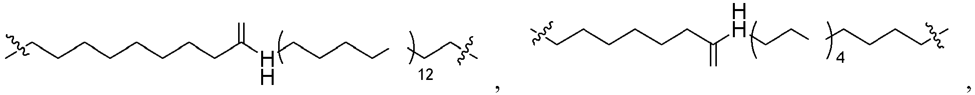

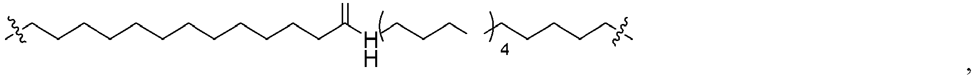

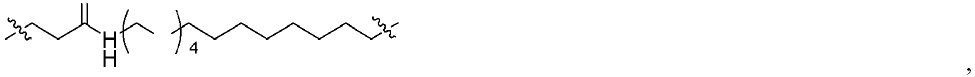

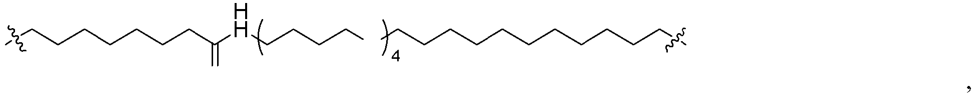

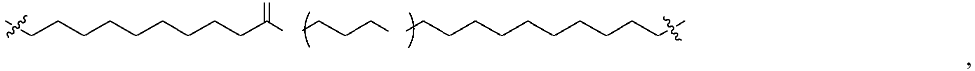

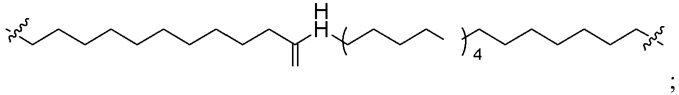

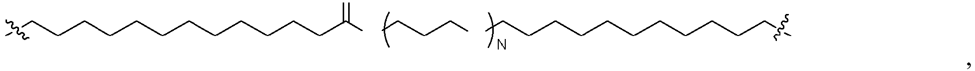

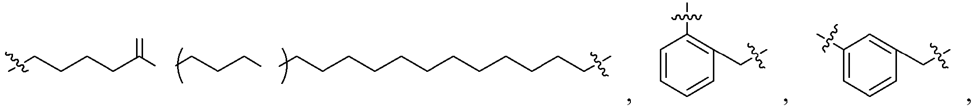

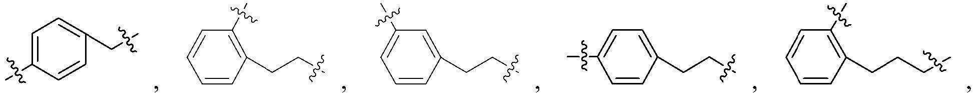

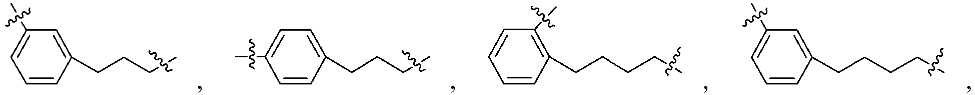

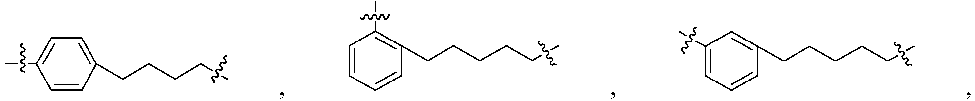

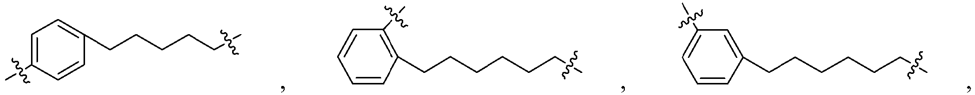

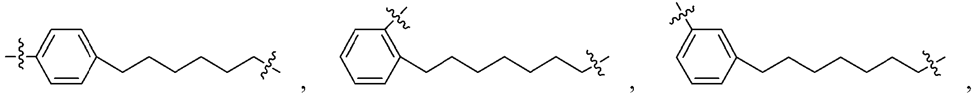

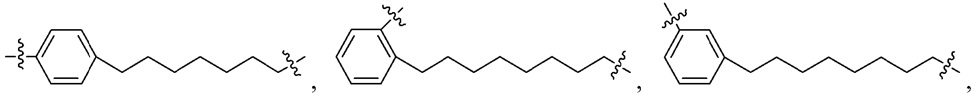

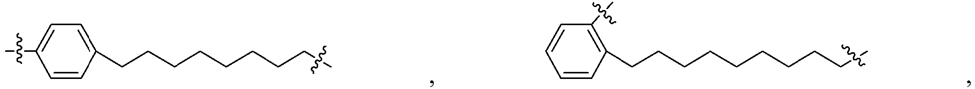

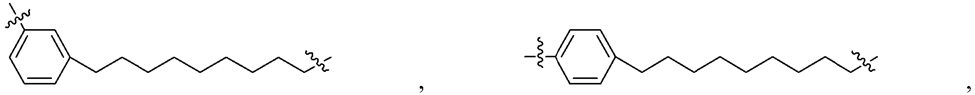

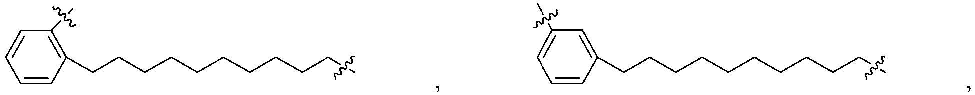

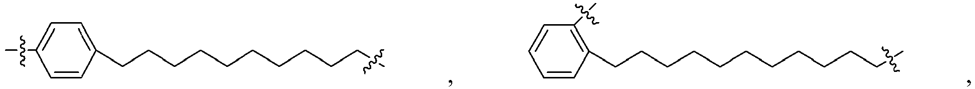

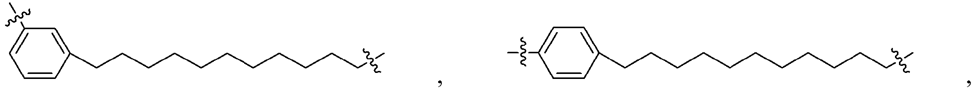

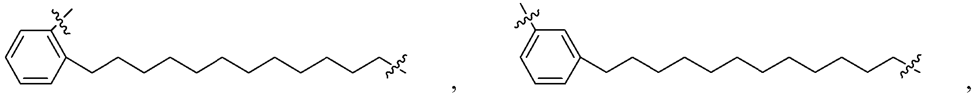

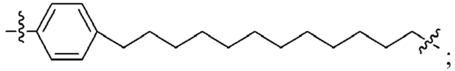

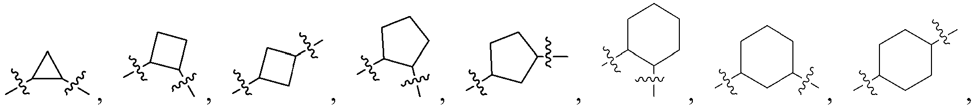

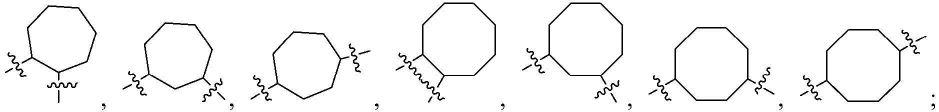

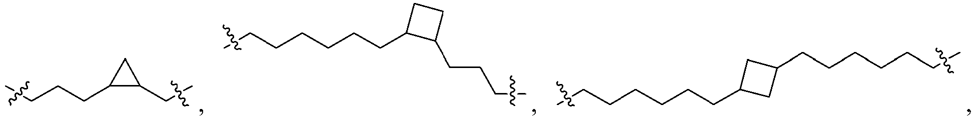

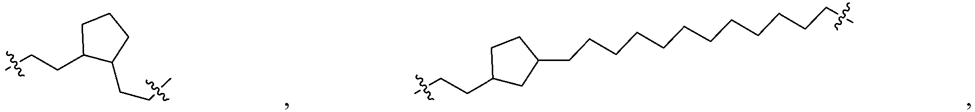

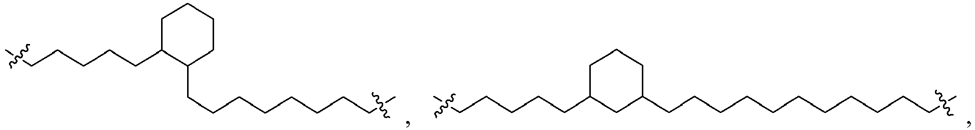

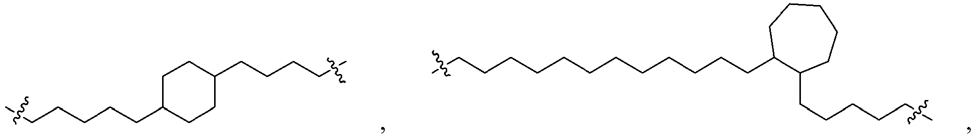

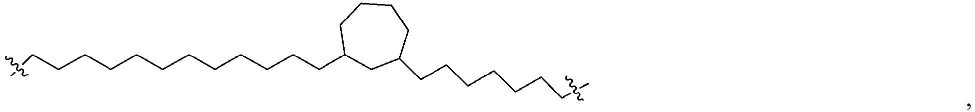

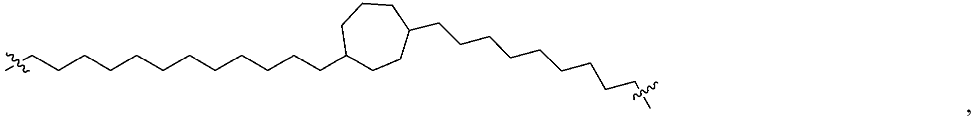

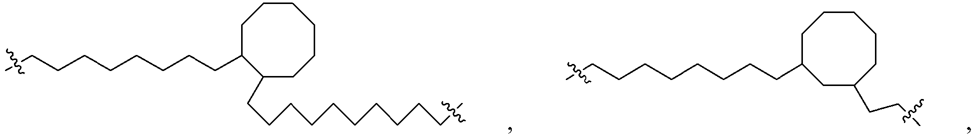

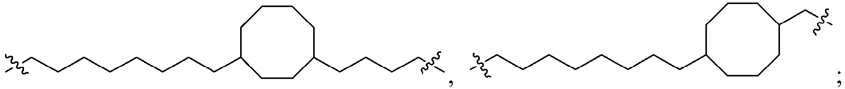

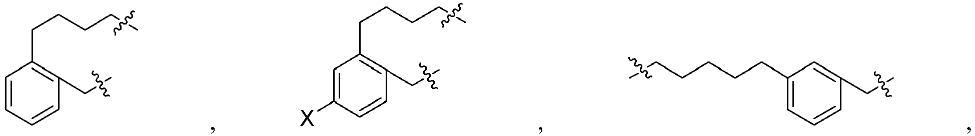

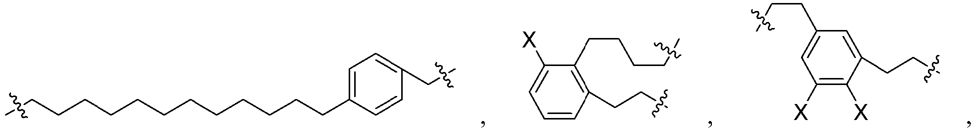

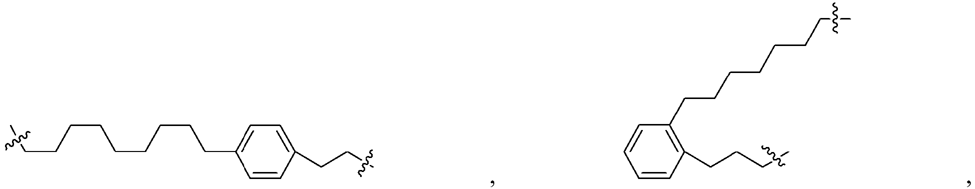

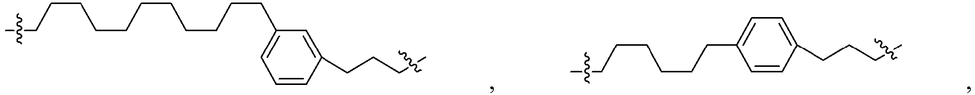

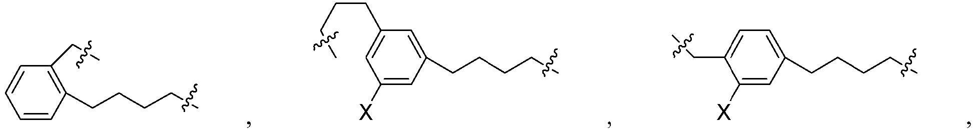

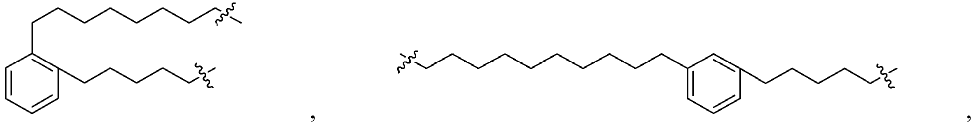

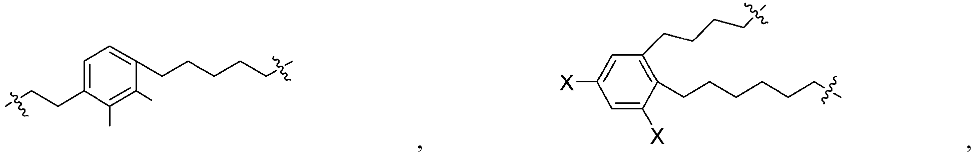

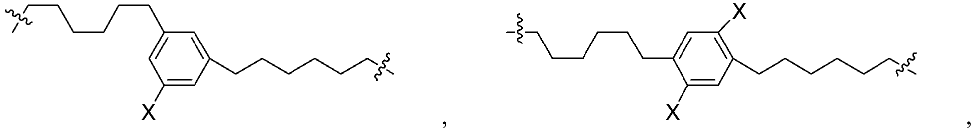

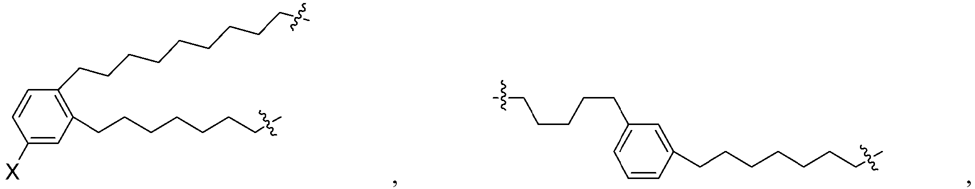

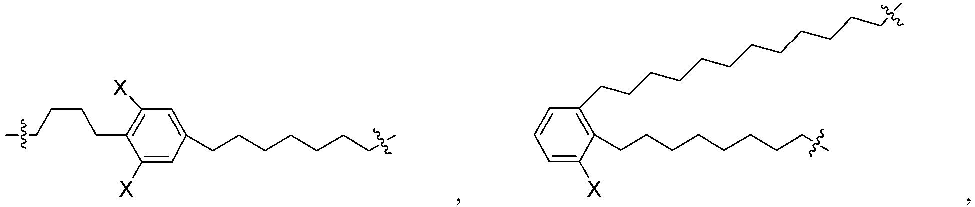

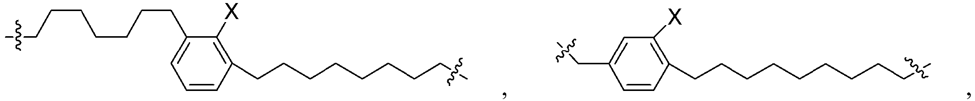

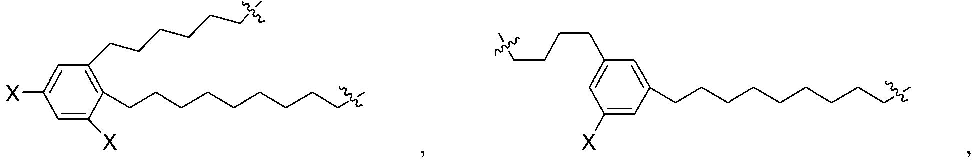

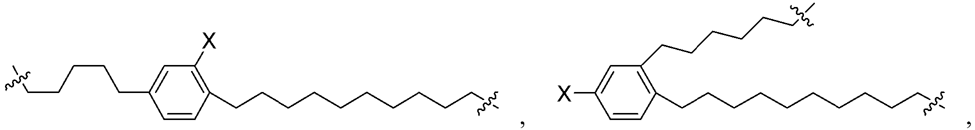

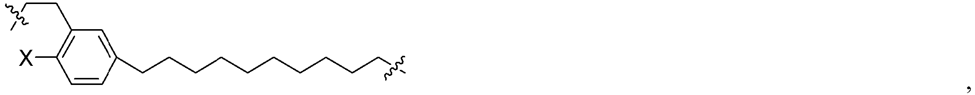

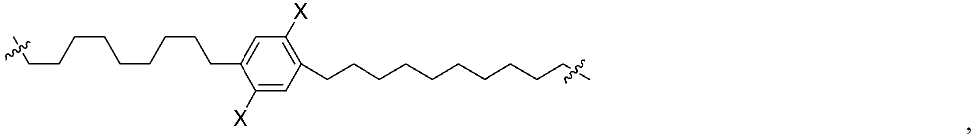

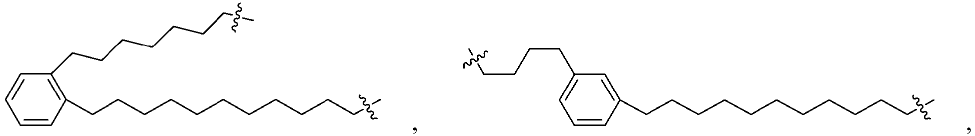

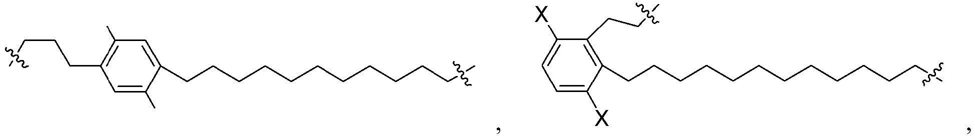

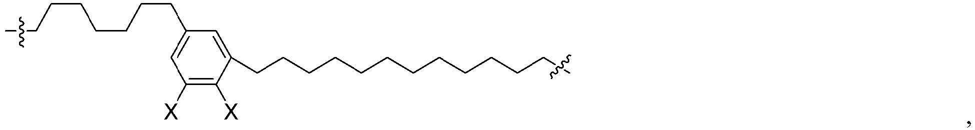

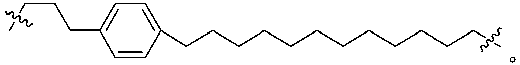

Specifically, the X₁ is selected from lysine, Lys, or glutamic acid, Glu;
when the X₁ is the lysine, a structure of the X₁ is 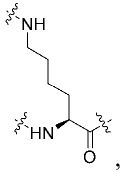 wherein a carboxyl terminus of the X₁ is connected to the A, and two amino termini of the X₁ are connected in a one-to-one correspondence to a side chain containing n1 or a side chain containing n2, that is, the side chain containing n1 can be connected to an amino group on a lysine main chain or can be connected to an amino group on a lysine side chain; and the side chain containing n2 can be connected to the amino group on the lysine main chain or can be connected to the amino group on the lysine side chain;
when the X₁ is the glutamic acid, Glu, a structure of the X₁ is 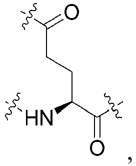 wherein an amino terminus is connected to the side chain containing n1 or the side chain containing n2; a carboxyl terminus adjacent to an amino group on the glutamic acid is connected to the A, and a carboxyl terminus remote from the amino group on the glutamic acid is connected to the side chain containing n1 or the side chain containing n2.

Specifically, the A is -Ala-Ala-Asn-, wherein an Ala terminus is connected to the lysine side chain, and an Asn terminus is connected to the C.

Specifically, the C is 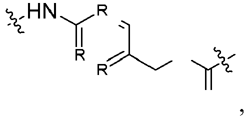 wherein R₂₃, R₂₄, and R₂₅ are CH or N, an amino terminus of the C is connected to the Asn, and a carbonyl terminus of the C is connected to the D.

Preferably, C includes, but is not limited to, the following structures:

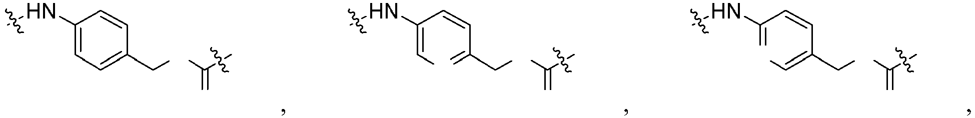

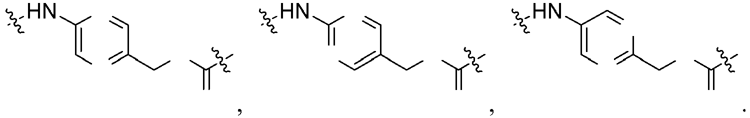

Specifically, the drug molecule D is one of the following chemical formulas:

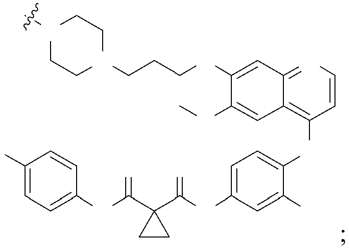

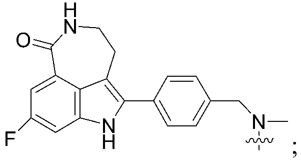

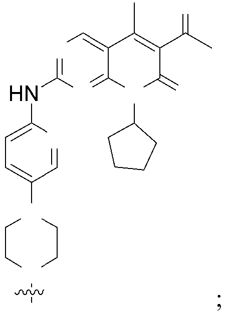

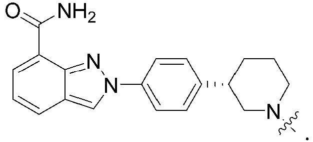

After the aforementioned drug molecule enters a human body and is cleaved by legumain, formed compound structures are as follows:

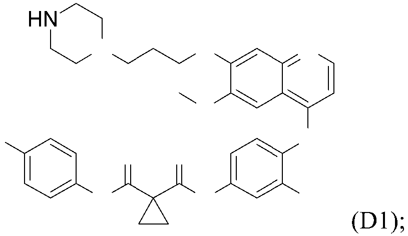

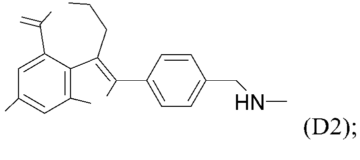

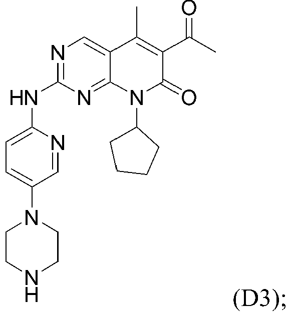

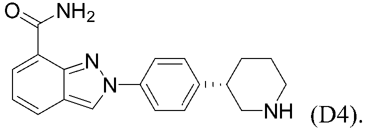

Chemical structures of representative drugs provided in the present invention are shown in Table 1:

**Table 1: Structures of kinase inhibitor compounds based on a tumor microenvironment**

| Name | Compound structure |
|---|---|
| Compound S1 | 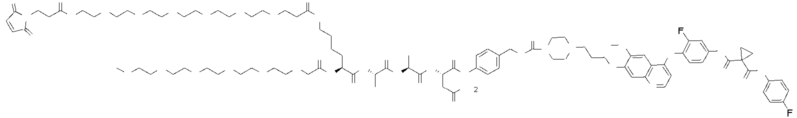 |
| Compound S2 | 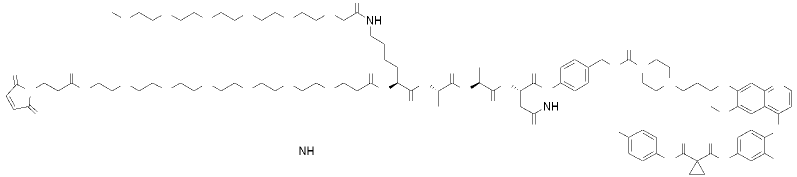 |
| Compound S3 | 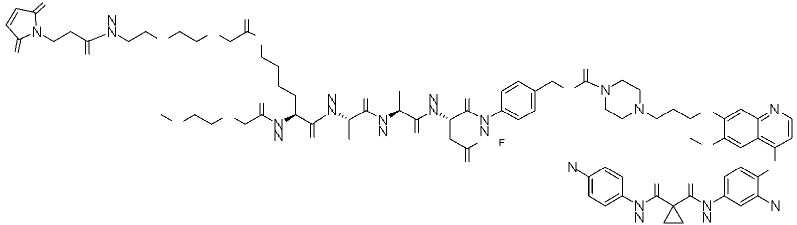 |
| Compound S4 | 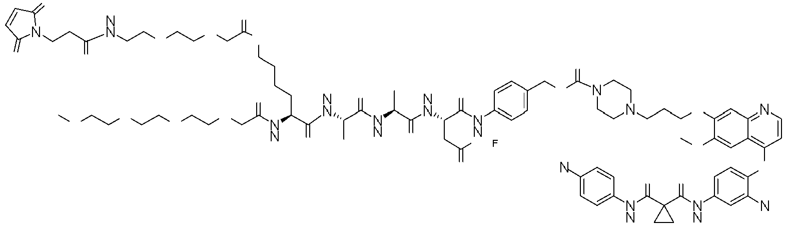 |
| Compound S5 | 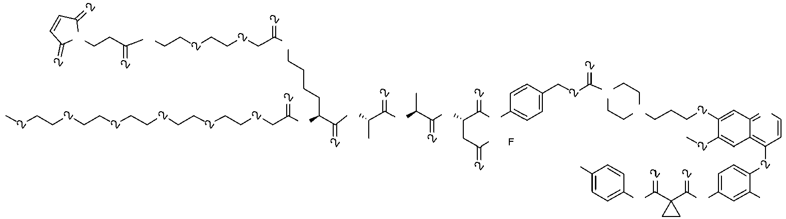 |
| Compound S6 | 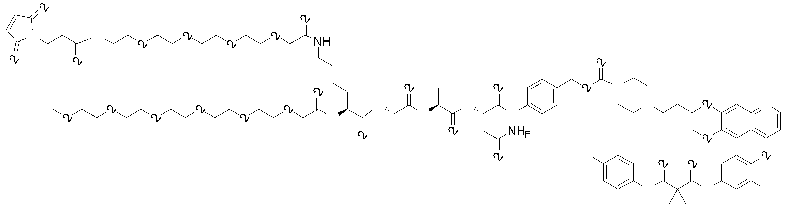 |
| Compound S7 | 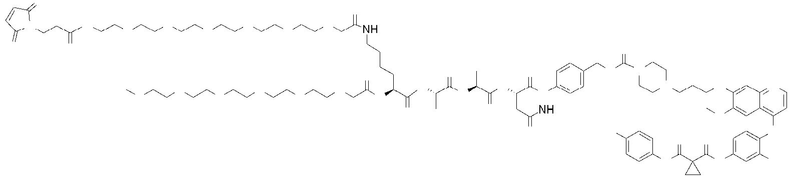 |
| Compound S8 | 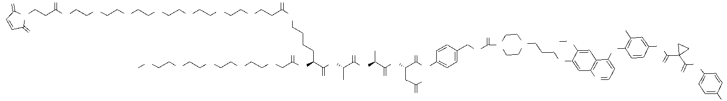 |
| Compound S9 | 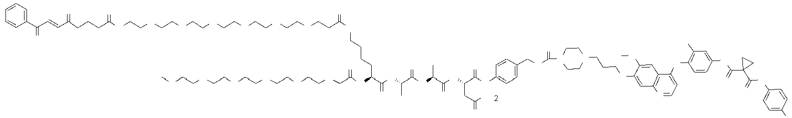 |
| Compound S10 | 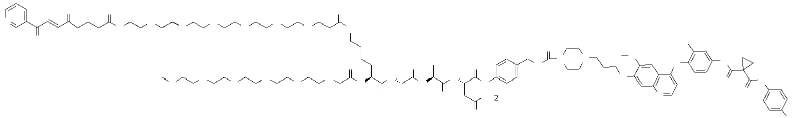 |
| Compound S11 | 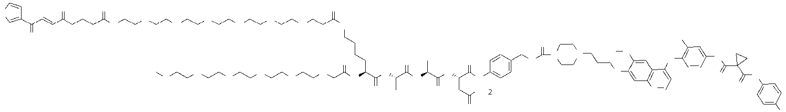 |
| Compound S12 | 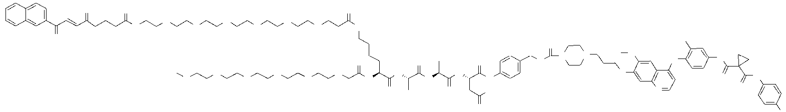 |
| Compound S13 | 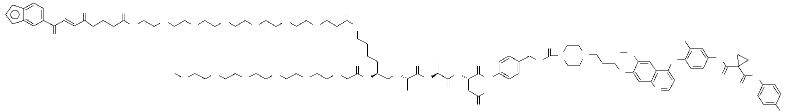 |
| Compound S14 | 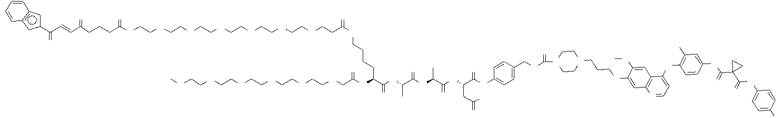 |
| Compound S15 | 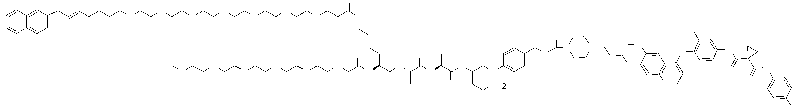 |
| Compound S16 | 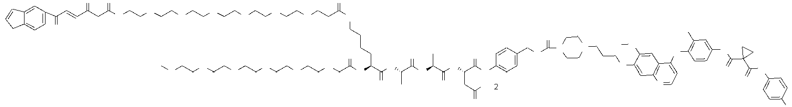 |
| Compound S17 | 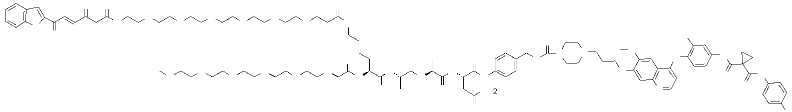 |
| Compound S18 | 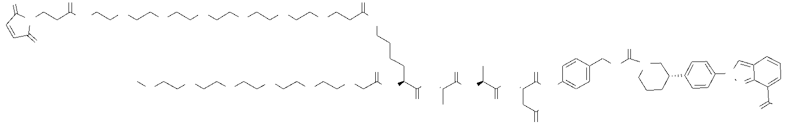 |
| Compound S19 | 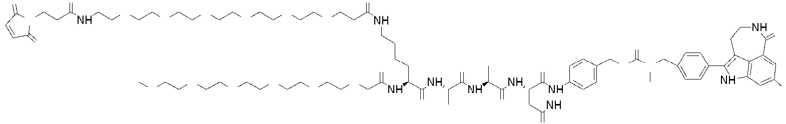 |
| Compound S20 | 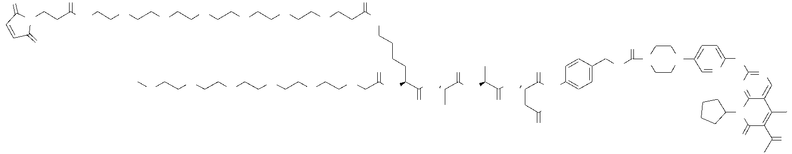 |
| Compound S21 | 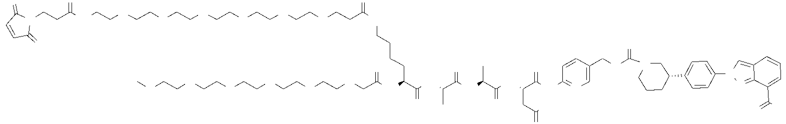 |
| Compound S22 | 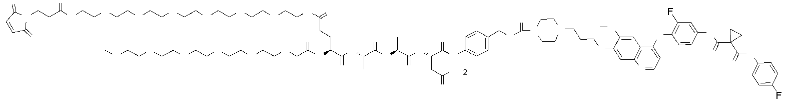 |
| Compound S23 | 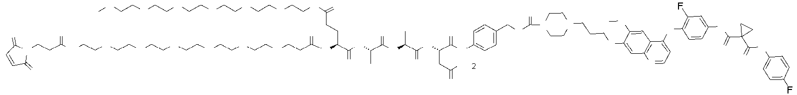 |

The aforementioned compounds are only an enumeration of some compound structures in the present invention. Compound structures involved in the summary of invention provided in the present invention are all within the protection scope of the present invention and are not enumerated repeatedly herein.

The present invention further provides comparative compounds as shown in the following table:

| Name | Compound structure |
|---|---|
| Compound C1 | 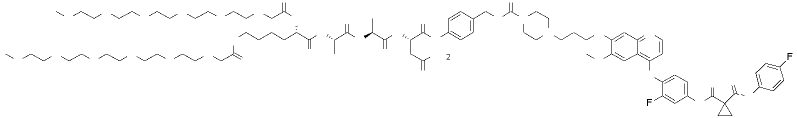 |

The comparative compound C1 is a compound without an MI group structure, and will be compared with the compound provided in the present invention in a subsequent experimental process.

### Embodiment 1: Synthesis of Compound S1

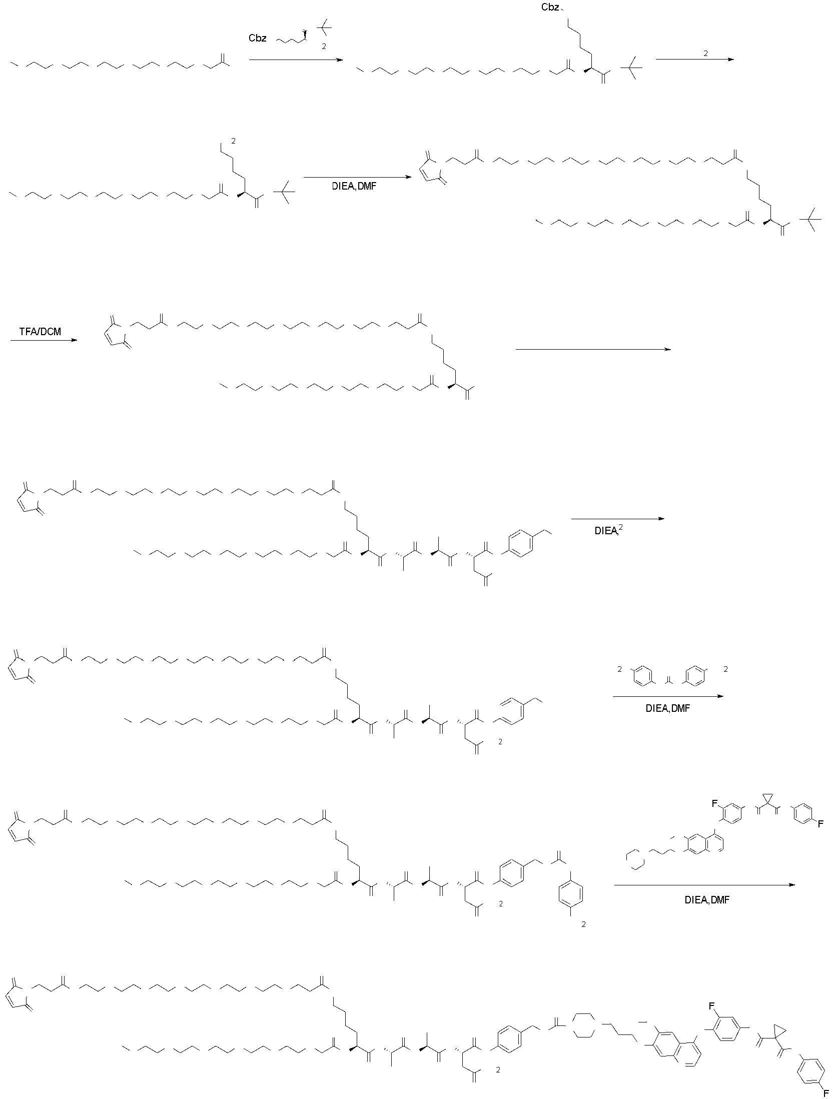

1. Pentaethylene glycol monomethyl ether acetic acid (Compound S1-1) (10 g, 32.2 mmol), HBTU (12.2 g, 32.2 mmol), DIEA (12.5 g, 96.6 mmol), and tert-butyl (S)-2-amino-6-(((benzyloxy)carbonyl)amino)hexanoate (11.9 g, 35.4 mmol) were dissolved in DMF (200 mL). The mixture was stirred at room temperature for 2 h. Completion of the reaction was determined by a TLC plate. 800 mL water was added to a reaction solution, and the reaction solution was extracted twice with ethyl acetate. An organic phase was washed with water, then washed with saturated brine, dried over anhydrous sodium sulfate, and concentrated under reduced pressure to dryness. The organic phase was purified by silica gel column (DCM:MeOH = 20:1) to obtain Compound S1-2 (18 g, 88.76 %) as a colorless oily liquid. LCMS: 629.4 (M+H)⁺.
2. Compound S1-2 (18 g, 28.6 mmol) was dissolved in 300 mL of ethyl acetate, 1.8 g of palladium on carbon was added, and the mixture was purged with hydrogen 3 times. The mixture was stirred at room temperature overnight, followed by suction filtration through diatomaceous earth and washing with ethyl acetate. An organic phase was concentrated under reduced pressure to dryness to obtain S1-3 (14 g, 98.8 %) as a colorless oily liquid. The compound was used directly for a next step reaction without purification. LCMS: 495.3 (M+H)⁺.
3. Compound S1-3 and DIEA (4.38 g, 34 mmol) were dissolved in DMF (200 mL), and 2,5-dioxopyrrolidin-1-yl1-(2,5-dioxo-2,5-dihydro-1H-pyrrol-1-yl)-3-oxo-7,10,13,16,19,22-hexao xa-4-azapentacosan-25-oate (17 g, 28.3 mmol) was added. The mixture was stirred at room temperature for 4 h. 800 mL water was added to a reaction solution, and the reaction solution was extracted twice with ethyl acetate. An organic phase was washed with water, then washed with saturated brine, dried over anhydrous sodium sulfate, and concentrated under reduced pressure to dryness. The organic phase was purified by silica gel column (DCM:MeOH = 10:1) to obtain S1-4 (22 g, 79.2 %) as a colorless oily liquid. LCMS: 981.5(M+H)⁺.
4. Compound S1-4 (22 g, 22.4 mmol) was dissolved in 150 mL of dichloromethane solution, 50 mL of trifluoroacetic acid was added, and a reaction solution was stirred at room temperature for 2 h. A solvent was concentrated under reduced pressure to dryness to obtain a crude product S1-5 (22 g) as a yellow oily liquid. LCMS: 925.5 (M+H)⁺.
5. S1-5 (22 g, 23.8 mmol), HBTU (9 g, 23.8 mmol), DIEA (9.2 g, 71.4 mmol), and H-Ala-Ala-Asn(Trt)-PABC-OH (14.7 g, 23.8 mmol) were dissolved in DMF (250 mL). The mixture was stirred at room temperature for 2 h. A solvent was concentrated under reduced pressure to dryness by an oil pump. The mixture was purified by silica gel column (DCM:MeOH = 10:1) to obtain S1-6 (15 g, 41.2 %) as a yellow solid. LCMS: 1528.4 (M+H)⁺.
6. S1-6 (15 g, 9.8 mmol) was dissolved in a 95 % aqueous trifluoroacetic acid solution and stirred at room temperature for 2 h. A solvent was concentrated under reduced pressure to dryness by an oil pump, then the mixture was dissolved in 80 mL of tert-butanol, and DIEA (6.3 g, 49 mmol) was added. The mixture was stirred at room temperature overnight. A solvent was concentrated under reduced pressure to dryness by an oil pump. The mixture was purified by silica gel column (DCM:MeOH = 4:1) to obtain S1-7 (5.5 g, 43.6 %) as a pale yellow solid. LCMS: 1286.4 (M+H)⁺.
7. S1-7 (5.5 g, 4.3 mmol) and bis(p-nitrophenyl) carbonate (3.9 g, 12.9 mmol) were dissolved in 70 mL DMF, DIEA (2.8 g, 21.5 mmol) was added thereto, and a reaction was performed at room temperature overnight. Purification by silica gel column with 15 % methanol/dichloromethane was performed to obtain S1-8 (3.8 g, yield 60.9 %) as a colorless oily liquid. LCMS: 1451.4 (M+H)⁺.
8. S1-8 (500 mg, 0.35 mmol) and S1-9 (221 mg, 0.35 mmol) were dissolved in 8 mL DMF, DIEA (90 mg, 0.7 mmol) was added thereto, and a reaction was performed at room temperature for 2 hours. Purified by preparative reverse-phase and lyophilized to obtain Compound S1 (350 mg, yield 51.5 %) as a white solid. LCMS: 1945.1 (M+H)⁺.

### Embodiment 2: Synthesis of Compound S2

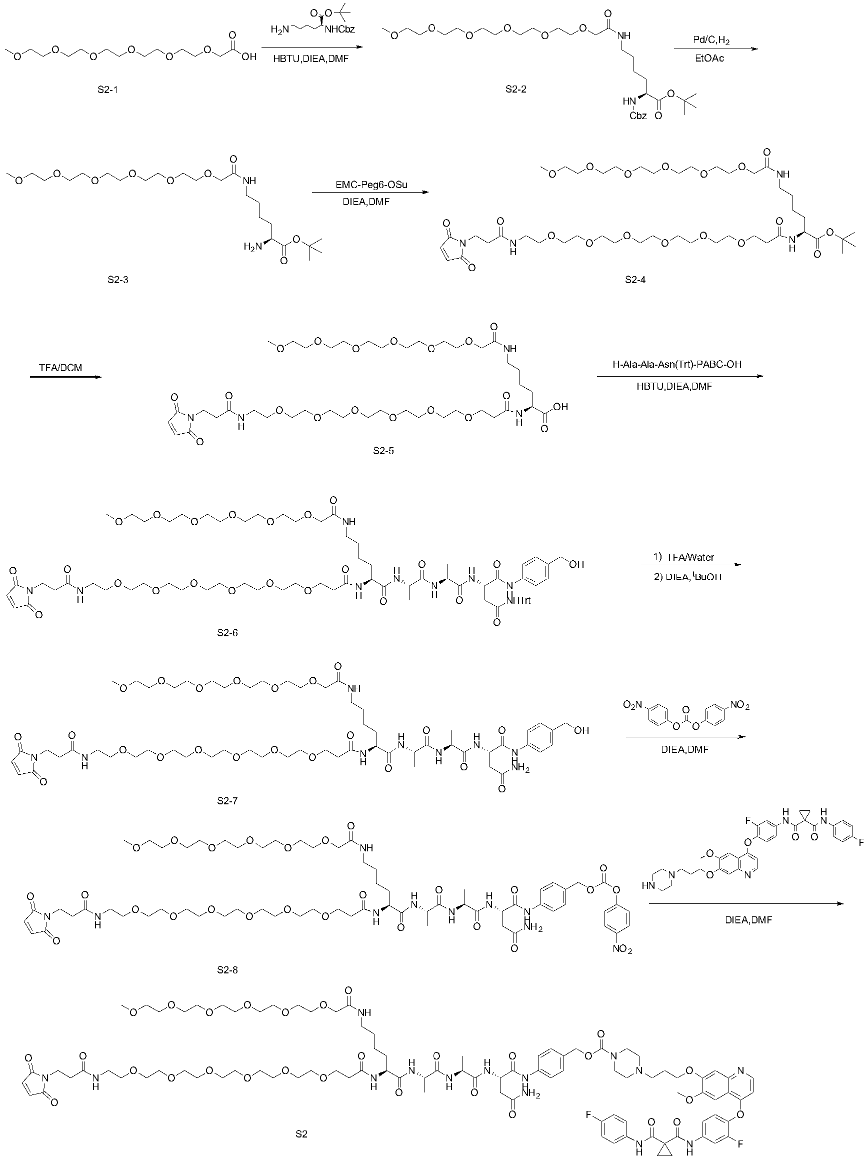

Referring to Embodiment 1, a white solid compound S2 (370 mg, a yield of 54.4 %) was obtained. LCMS: 1945.1 (M+H)⁺.

Synthetic routes and methods for compounds S3-S23 provided in the present invention were performed by referring to Embodiment 1 and Embodiment 2. The compounds S3-S8 are products synthesized by adjusting a length of a peg chain based on Embodiments 1 and 2; the compounds S9-S17 are products synthesized by selecting different groups for an MI structure based on Embodiments 1 and 2; the compounds S18-S20 are products generated by substitution of different drug molecules D; S21 is a heteroatom substitution product of PABC; and S22 and S23 are products generated by selecting an X₁ group from glutamic acid based on Embodiments 1 and 2. Synthesis methods for the compounds S3-S23 are similar to Embodiment 1 and Embodiment 2, and are not described repeatedly herein.

### Embodiment 3: Synthesis of Compound S18

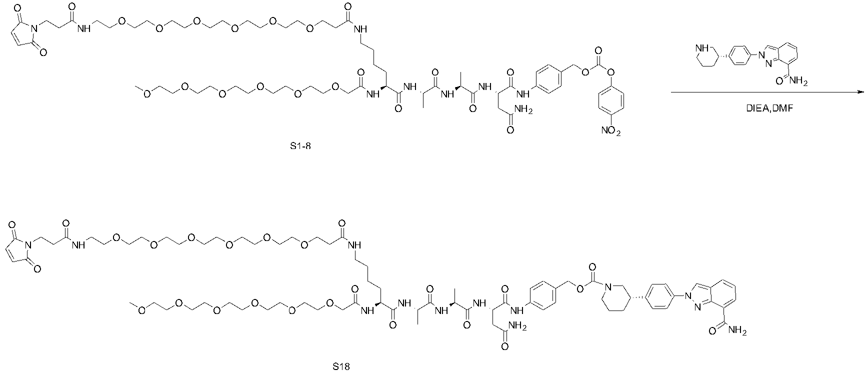

The compound S1-8 and niraparib (221 mg, 0.69 mmol) were dissolved in 15 mL DMF, and DIEA (178 mg, 1.38 mmol) was added thereto. A reaction was performed at room temperature for 2 hours. Purified by preparative reverse-phase and lyophilized to obtain a white solid compound S18 (330 mg, a yield of 29.3 %). LCMS: 1632.8 (M+H)⁺.

### Embodiment 4: Synthesis of Compound S19

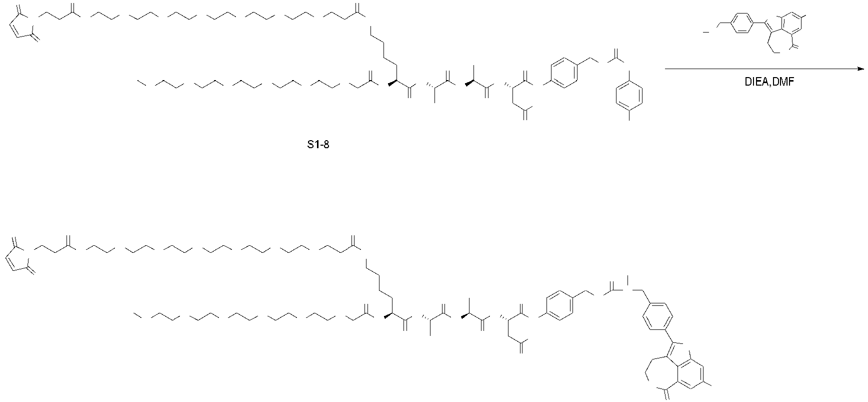

The compound S1-8 (1 g, 0.69 mmol) and rucaparib (223 mg, 0.69 mmol) were dissolved in 15 mL DMF, and DIEA (178 mg, 1.38 mmol) was added thereto. A reaction was performed at room temperature for 2 hours. Purified by preparative reverse-phase and lyophilized to obtain a pale yellow solid compound S19 (300 mg, a yield of 26.6 %). LCMS: 1635.8 (M+H)⁺.

### Embodiment 5: Legumain enzymatic cleavage assay of the compound

### 1.1 Solution preparation

Mobile phase A (0.1 % TFA /H2O): 1 mL TFA was added to 1000 mL water, and a mixture was mixed uniformly and then ultrasonicated for 10 minutes for use.

Mobile phase B (ACN): 1000 mL acetonitrile was ultrasonicated for 10 minutes for use.

Test buffer: 50 mM MES, 250 mM sodium chloride, pH was adjusted to 5.0.

Legumain: 1 mg/mL.

### 1.2 Preparation of a sample solution

Preparation of a sample solution: Appropriate amounts of 23 compound samples in Table 1 were weighed into sample tubes, DMSO was added to dissolve the samples to 4 mM, and water was added to dilute the samples to 0.5 mM.

### 1.3 Enzymatic cleavage assay:

The present assay includes a control group and a Legumain enzymatic cleavage group.

The sample solution and the test buffer were added into a sample tube at a volume ratio of 1:3 (90 µL buffer was added to 30 µL sample solution) and mixed uniformly. At different time points (0 h, 5 min, 1 h, 2 h, 4 h, 6 h, 20 h), a residual amount of a sample was detected by HPLC as a control, and an injection volume was 10 µL.

The sample solution, Legumain, and the test buffer were added into a sample tube at a volume ratio of 1:1:2 (30 µL Legumain and 60 µL buffer were added to 30 µL sample solution) and mixed uniformly. At different time points (0 h, 5 min, 1 h, 2 h, 4 h, 6 h, 20 h), the residual amount of the sample was detected by HPLC, and an enzymatic cleavage product was compared, and the injection volume was 10 µL.

### 2.1 Results of enzymatic cleavage

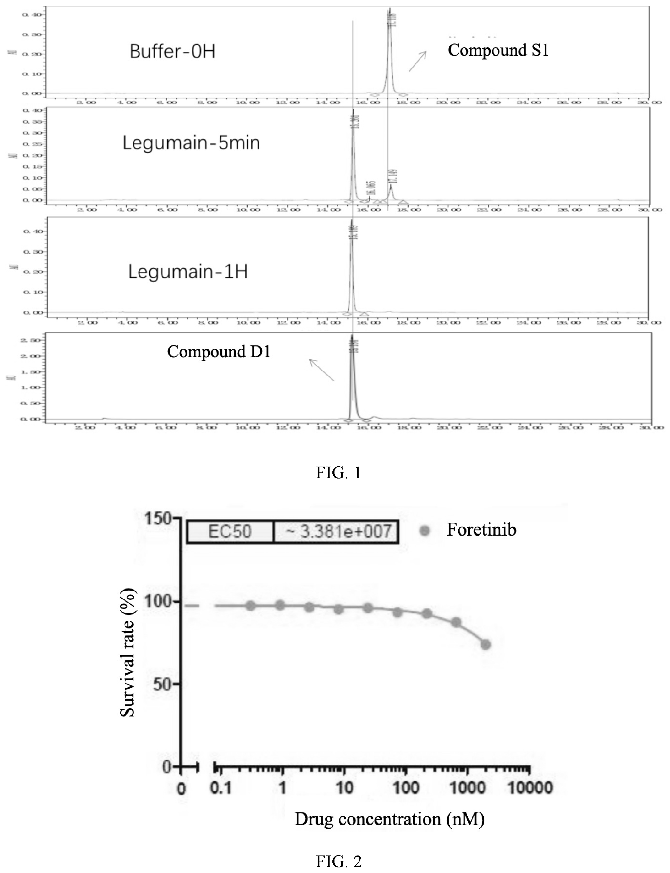

As shown in FIG. 1, which is a chromatogram of Legumain enzymatic cleavage of the sample compound S1, a result indicates that at 1 h, the compound S1 was completely cleaved under an action of Legumain enzyme to obtain a compound D1 product. HPLC chromatograms at subsequent 2 h, 4 h, 6 h, and 20 h are the same as a result of 1 h in FIG. 1, and are not shown or described repeatedly herein. By using a same method, the aforementioned Legumain enzymatic cleavage assay was also performed on the compounds provided in the present invention. Results indicate that under the aforementioned conditions, other 22 compounds provided in Table 1 could all be completely cleaved by Legumain within 1 h to completely release a corresponding small molecule drug. It is indicated that structures similar to the 23 compounds provided in the present invention can all be cleaved by Legumain in vivo and release corresponding small molecule drugs, thereby performing treatment after targeting tumor cells.

Results of the present enzymatic cleavage assay show that enzymatic cleavage efficiency at 1 h of the compounds S1-S23 in Table 1 of the present invention all reached 100 %. The efficiency is significantly higher than that of a compound disclosed in the prior art (such as the compound S1 disclosed in Chinese invention patent CN104262455B, which serves as a control compound S0 of the present invention, and a structure thereof is shown as the following formula). It is known that enzymatic cleavage of the compound S0 at 2 h is 80 %, and the enzymatic cleavage efficiency of the compound S0 is lower than that of the compounds S1-S23 of the present invention.

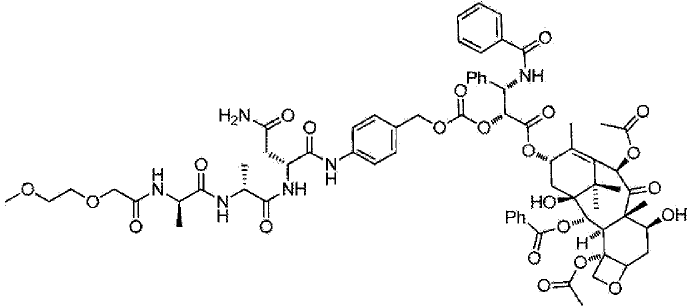

### Control compound S0

### Embodiment 6: Solubility assay of the compound:

5.0 mg of each of the 23 compounds in Table 1 was precisely weighed, and 0.25 mL sterile water for injection was added for dissolution until the compound was completely dissolved into a clear solution. No change occurred after being placed at room temperature for 8 hours. Solubilities of the 23 compounds obtained through calculation are shown in the following Table 2.

**Table 2: Solubilities of Compound S1-Compound S23**

| Compound No. | Solubility (mg/mL) |
|---|---|
| S 1 | 50 |
| S 2 | 48 |
| S 3 | 20 |
| S 4 | 22 |
| S 5 | 25 |
| S 6 | 29 |
| S 7 | 45 |
| S 8 | 40 |
| S 9 | 39 |
| S 10 | 41 |
| S 11 | 40 |
| S 12 | 32 |
| S 13 | 38 |
| S 14 | 36 |
| S 15 | 34 |
| S 16 | 37 |
| S 17 | 36 |
| S 18 | 44 |
| S 19 | 43 |
| S 20 | 46 |
| S 21 | 45 |
| S 22 | 48 |
| S 23 | 47 |

### Embodiment 7: Stability test of the compound

According to the compounds shown in Table 1, 23 groups of compounds were precisely weighed with 2.0 mg for each group. A 0.1 mol/L citrate buffer (pH was 4.5, 5.0, 5.5, 6.0, 6.5, 7.0, and 7.4, respectively) was added to dissolve the compounds to a 0.5 mmol/L clear solution. Sampling was performed to detect chemical stability of samples at 0 h, 4 h, and 8 h under different pH conditions. After the samples were placed under different pH conditions for 30 min, detection was performed by using HPLC and LCMS. Results showed that the samples basically had no change under a condition of being placed in a neutral or weakly acidic environment for 8 h. Under a condition of pH 7.4 for 8 h, a small number of impurities appeared in the samples, which were detected to be a maleimide ring-opening product. The aforementioned stability test results indicate that compound samples having structures similar to the 23 compounds in Table 1 provided in the present invention are very stable under neutral or weakly acidic conditions.

### Embodiment 8: Analysis of inhibitory activities of compound D1, foretinib, and staurosporine on kinase by ADP-Glo method

1) 1 × Kinase buffer was prepared. 1) Preparation of a compound concentration gradient: A starting test concentration of a test compound was 10000 nM, with 3-fold dilution, 10 concentrations, and a single-well test. 50 nL was transferred by Echo to compound wells of a 384-well plate; 50 nL of DMSO was added to negative control wells and positive control wells, respectively.
2) A kinase solution with 2 times a final concentration was prepared with the 1 × Kinase buffer.
3) 2.5 µL of the kinase solution with 2 times the final concentration was added to the compound wells and the positive control wells, respectively; 2.5 µL of the 1 × Kinase buffer was added to the negative control wells.
4) Centrifugation was performed at 1000 rpm for 30 seconds. After shaking and mixing uniformly, the mixture was incubated at room temperature for 60 minutes.
5) A mixed solution of ATP and a substrate Fluorescein-MAP2Kl with 2 times a final concentration was prepared with the 1 × Kinase buffer.
6) 2.5 µL of the mixed solution of ATP and the substrate with 2 times the final concentration was added to initiate the reaction.
7) The 384-well plate was centrifuged at 1000 rpm for 30 seconds. After shaking and mixing uniformly, the mixture was incubated at room temperature for 120 minutes.
8) 5 µL of ADP-Glo Reagent was added, followed by centrifugation at 1000 rpm for 30 seconds. After shaking and mixing uniformly, the mixture was incubated at room temperature for 180 minutes.
9) 10 µL of Kinase Detection Reagent was added, followed by centrifugation at 1000 rpm for 30 seconds. After shaking and mixing uniformly, the mixture was incubated at room temperature for 30 minutes.
10) A luminescence value RLU was read by an Envision microplate reader.
11) Calculation formula for the ADP-Glo method: $Inhibition % = 100 - \frac{RLU - Mean \left(NC\right)}{Mean \left(PC\right) - Mean \left(NC\right)} \times 100$
   (wherein, RLU: a chemiluminescence value of a sample; Mean (NC): an average value of ratios of negative control wells; Mean (PC): an average value of ratios of positive control wells.)

### III. Experimental results

Experimental results obtained through the aforementioned experimental process are shown in Table 3 below:

**Table 3: Analysis results of IC₅₀ of inhibitory activities of compound D1, foretinib, and staurosporine on kinase by ADP-Glo method**

| Compound | Met (nM) | KDR (VEGFR2) (nM) | AXL (nM) | FLT3 (nM) | Kit (nM) |
|---|---|---|---|---|---|
| Foretinib | 16.7 | 4.87 | 0.71 | 30 | 7.8 |
| Compound D1 | 9.13 | 2.34 | 0.23 | 7.5 | 9.1 |
| Staurosporine | 92 | 5.22 | 1.42 | 0.19 | 3.4 |

According to Table 3, inhibitory activity of foretinib on AXL is within 1 nM, and inhibitory activity of staurosporine on FLT3 is within 1 nM, which are consistent with the kinase selectivity of foretinib and staurosporine reported in literature. Activities of the compound D1 on kinases Met and VEGFR are significantly better than those of a foretinib molecule. In particular, the compound D1 exhibits very high activity on the kinase AXL. Compared with the foretinib molecule, the activity of the compound D1 is significantly improved by one time.

### Embodiment 9: Cytotoxicity study of foretinib, compound D1, and compound S1 on B16F10 cells

### I. Experimental steps

1) B16F10 cells (B16F10 mouse skin melanoma cells) in a logarithmic phase were collected, and a concentration of a cell suspension was adjusted. 100 µL was added to each well. During seeding, a density of cells to be tested was adjusted to 5000 cells/well (peripheral wells were filled with sterile PBS).
2) Incubation was performed at 37 °C with 5 % CO2 until a cell monolayer covered a bottom of a well (96-well flat bottom plate). Drugs with a concentration gradient were added. A final concentration of the drugs was 0 to 100 µM, with a total of 7 concentration points. Fold dilution was performed, and 100 µL was added to each well. 3 replicate wells were set.
3) Incubation was performed at 37 °C with 5 % CO2 for 48 hours, followed by observation under an inverted microscope.
4) 20 µL of MTT solution (5 mg/mL, i.e., 0.5 % MTT) was added to each well, and culture was continued for 4 h. If a drug can react with MTT, culture medium can be discarded after centrifugation first. After being carefully washed with PBS for 2 to 3 times, a culture medium containing MTT was then added.
5) Culture was terminated, and the culture medium in the wells was carefully sucked away.
6) 150 µL of dimethyl sulfoxide was added to each well, and the plate was placed on a shaker for low-speed oscillation for 10 min to fully dissolve crystals. An absorbance value of each well was measured at OD 490 nm of a microplate reader.
7) Meanwhile, zeroing wells (medium, MTT, dimethyl sulfoxide) and control wells (cells, drug dissolution medium of a same concentration, culture medium, MTT, dimethyl sulfoxide) were set.
8) Calculation formula for inhibition rate: Inhibition rate = OD_ₛₐₘₚₗₑ - OD__{zeroing} well/OD__{control well} - OD__{zeroing well}.
9) By taking a log value of a concentration as an X-axis and a percentage inhibition rate as a Y-axis, a fitted dose-response curve was obtained using log(inhibitor) vs. response - Variable slope of analysis software GraphPad Prism 5, thereby obtaining IC50 values of each compound on enzyme activity.

### II. Experimental results

As shown in FIG. 2 to 4, inhibition curves of the compound D1 and foretinib are consistent, and toxicity of the compound D1 to B16F10 cells is stronger than that of foretinib. Moreover, toxicity of the compound S1 is lower than that of the compound D1 and foretinib. The aforementioned phenomenon indicates that the compound D1 released after the compound S1 targetedly enters tumor cells has a better therapeutic effect than existing drugs.

### Embodiment 10: Cytotoxicity study of compound S1, foretinib, and compound D1 on A549 cells

### I. Experimental steps

1) A549 cells (A549 cells are lung cancer human alveolar basal epithelial cells) in a logarithmic phase were collected, and a concentration of a cell suspension was adjusted. 100 µL was added to each well. During seeding, a density of cells to be tested was adjusted to 5000 cells/well (peripheral wells were filled with sterile PBS).
2) Incubation was performed at 37 °C with 5 % CO2 until a cell monolayer covered a bottom of a well (96-well flat bottom plate). Drugs with a concentration gradient were added. A final concentration of the drugs was 0 to 100 µM, with a total of 7 concentration points. Fold dilution was performed, and 100 µL was added to each well. 3 replicate wells were set.
3) Incubation was performed at 37 °C with 5 % CO2 for 48 hours, followed by observation under an inverted microscope.
4) 20 µL of MTT solution (5 mg/mL, i.e., 0.5 % MTT) was added to each well, and culture was continued for 4 h. If a drug can react with MTT, culture medium can be discarded after centrifugation first. After being carefully washed with PBS for 2 to 3 times, a culture medium containing MTT was then added.
5) Culture was terminated, and the culture medium in the wells was carefully sucked away.
6) 150 µL of dimethyl sulfoxide was added to each well, and the plate was placed on a shaker for low-speed oscillation for 10 min to fully dissolve crystals. An absorbance value of each well was measured at OD 490 nm of a microplate reader.
7) Meanwhile, zeroing wells (medium, MTT, dimethyl sulfoxide) and control wells (cells, drug dissolution medium of a same concentration, culture medium, MTT, dimethyl sulfoxide) were set.
8) Calculation formula for inhibition rate: Inhibition rate = OD_ₛₐₘₚₗₑ - OD__{zeroing well}/OD__{control well} - OD__{zeroing well}.
9) By taking a log value of a concentration as an X-axis and a percentage inhibition rate as a Y-axis, a fitted dose-response curve was obtained using log(inhibitor) vs. response - Variable slope of analysis software GraphPad Prism 5, thereby obtaining IC50 values of each compound on enzyme activity.

### II. Experimental results

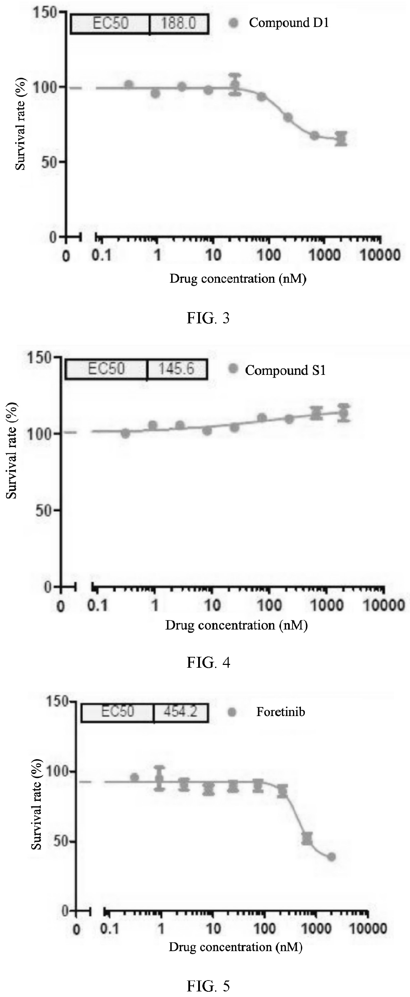

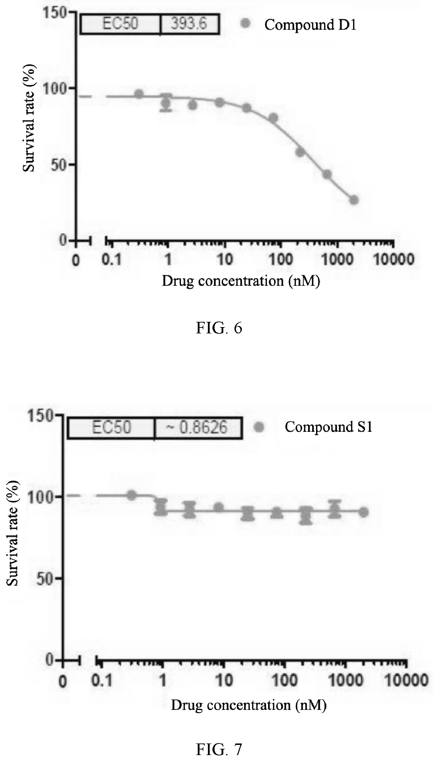

As shown in FIG. 5 to 7, toxicity of the compound D1 to A549 cells is stronger than that of foretinib. It is indicated that an effect of the compound S1 provided in the present invention on tumor cells is better than that of the drug foretinib used in the prior art, and the compound S1 has a high targeting effect.

Embodiment 9 and Embodiment 10 indicate that toxicities of the compound D1 and a similar structure thereof in B16F10 cell and A549 cell models are both stronger than that of foretinib. Moreover, an effect of the compound S1 on tumor cells in the aforementioned two cell models is better than that of foretinib used in the prior art. Damage to normal cells is reduced, and a targeting effect of treatment is improved.

### Embodiment 11: Efficacy study of compound S1 and combination of compound S1 with Anti-mPD-1 in treatment of CT-26 tumor model

### I. Experimental materials

Test substances: control compound C1, a low dose of 3.95 µmol/kg, a medium dose of 7.90 µmol/kg, and a high dose of 47.42 µmol/kg; compound S1, a low dose of 3.95 µmol/kg, a medium dose of 7.90 µmol/kg, and a high dose of 47.42 µmol/kg; and Anti-mPD-1.

Test animals: Balb/C mice, 7 to 8 weeks of age, female, obtained from Shanghai Lingchang Biotechnology Co., Ltd.

### II. Preparation of tumor model:

1) Cell culture: CT-26 cells were obtained from the Cell Bank of the Chinese Academy of Sciences. The cells were cultured in RPMI-1640 medium containing 10 % fetal bovine serum in a 37 °C, 5 % CO2 incubator. Passage was performed once every three days, and cells within 15 passages were used.
2) Cell inoculation: A concentration of cells was adjusted to 3.0 × 10⁶/mL with RPMI-1640 basic medium. The cells were inoculated subcutaneously into a right back of the mice, 0.1 mL per mouse.
3) Treatment: When a tumor volume reached 100 mm³ to 200 mm³, the mice were randomly divided into 11 groups and treatment was started. 6 mice were included in each group, which were respectively a normal saline negative control group, a foretinib positive control group, a compound D1 control group, a control compound C1 low dose group, a control compound C1 medium dose group, a control compound C1 high dose group, a compound S1 low dose group, a compound S1 medium dose group, a compound S1 high dose group, an Anti-mPD-1 single-use group, and a compound S1 and Anti-mPD-1 combined treatment group. A day of grouping was set as D0, and experimental results were D20 (i.e., results of the 20th day).

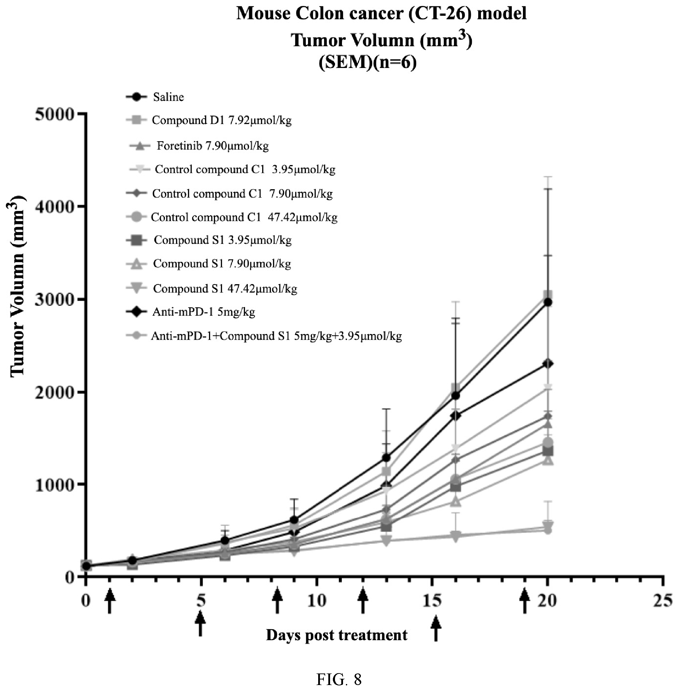

As shown in FIG. 8 and Table 4, which show an effect of test substances on growth of mouse xenograft CT-26.

**Table 4: Effects of corresponding compounds and control groups on tumor inhibition**

| Group | N | Tumor volume (mean ± SEM) (mm³) | Tumor growth inhibition rate TGI (%) | Body weight vs D0 (%) |
|---|---|---|---|---|
| | | D20 | D20 | D20 |
| Normal saline | 6 | 2967.71 + 1218.75 | - | 14.2% |
| Foretinib | 6 | 1656.25 ± 369.27 | 45.98% | 19.3% |
| Compound D1 | 6 | 3047.86 + 1271.21 | -2.83% | 13.4% |
| Control compound C1 3.95 µmol/kg | 6 | 2036.81 ± 883.75 | 32.69% | 8.2% |
| Control compound C1 7.90 µmol/kg | 6 | 1739.97 ± 596.04 | 42.99% | 11.6% |
| Control compound C1 47.42 µmol/kg | 6 | 1456.74 ± 254.88 | 52.93% | 9.9% |
| Compound S1 3.95 µmol/kg | 6 | 1366.65 ± 424.88 | 56.47% | 7.0% |
| Compound S1 7.90 µmol/kg | 6 | 1267.47 ± 269.49 | 59.91% | 17.8% |
| Compound S1 47.42 µmol/kg | 6 | 541.18 ± 68.71 | 85.06% | 3.1% |
| Anti-mPD-1 5 mg/kg | 6 | 2304.8 ± 1163.18 | 23.13% | 7.8% |
| Anti-mPD-1 + Compound S1 5 mg/kg + 3.95 µmol/kg | 6 | 501.78 ± 318.53 | 86.44% | 7.1% |

According to FIG. 8 and Table 4, in the CT-26 syngeneic xenograft model, compared with the normal saline group, foretinib, the control compound C1, and the compound S1 all exhibited good tumor inhibition effects and showed a dose-dependent effect, while the compound D1 did not exhibit a tumor inhibition effect. Compared with the foretinib positive control group, at an equimolar dose, both the control compound C1 and the compound S1 exhibited certain efficacy advantages, and an effect of the compound S1 is better than that of the compound C1.

### Embodiment 12: Efficacy study of compound S1, compound S2, compound S12, and compound S15 in treatment of MKN45 tumor model

### I. Experimental materials

Test substances: control compound C1, a low dose of 7.90 µmol/kg, a medium dose of 39.5 µmol/kg, and a high dose of 79 µmol/kg; compound S1, a low dose of 7.90 µmol/kg, a medium dose of 39.5 µmol/kg, and a high dose of 79 µmol/kg; compound S2, a low dose of 7.90 µmol/kg, a medium dose of 39.5 µmol/kg, and a high dose of 79 µmol/kg; compound S12, a low dose of 7.90 µmol/kg, a medium dose of 39.5 µmol/kg, and a high dose of 79 µmol/kg; compound S15, a low dose of 7.90 µmol/kg, a medium dose of 39.5 µmol/kg, and a high dose of 79 µmol/kg.

Test animals: NOD-SCID mice, 7 to 8 weeks of age, female, obtained from Nanjing GemPharmatech Co., Ltd.

### II. Preparation of tumor model:

1) Cell culture: MKN45 cells were obtained from the Cell Bank of the Chinese Academy of Sciences. The cells were cultured in RPMI-1640 medium containing 20 % fetal bovine serum in a 37 °C, 5 % CO₂ incubator. Passage was performed once every three days, and cells within 15 passages were used.
2) Cell inoculation: A concentration of cells was adjusted to 5.0 × 10⁷/mL with RPMI-1640 basic medium. The cells were inoculated subcutaneously into a right back of the mice, 0.1 mL per mouse.
3) Treatment: When a tumor volume reached 100 mm³ to 200 mm³, the mice were randomly divided into groups and treatment was started. 6 mice were included in each group, which were respectively: a normal saline negative control group, a foretinib positive control group, a compound D1 control group, a control compound C1 low dose group, a control compound C1 medium dose group, a control compound C1 high dose group, a compound S1 low dose group, a compound S1 medium dose group, a compound S1 high dose group, a compound S2 low dose group, a compound S2 medium dose group, a compound S2 high dose group, a compound S12 low dose group, a compound S12 medium dose group, a compound S12 high dose group, a compound S15 low dose group, a compound S15 medium dose group, and a compound S15 high dose group. A day of grouping was set as D0, and experimental results were D28 (i.e., results of the 28th day).

Administration results are shown in Table 5.

**Table 5: Effects of corresponding compounds and control groups on tumor inhibition**

| Group | N | Tumor volume (mean ± SEM) (mm³) | Tumor growth inhibition rate TGI (%) | Body weight vs D0 (%) |
|---|---|---|---|---|
| | | D36 | D28 | D28 |
| Normal saline | 6 | 1089.84 ± 154.98 | - | -5.0% |
| Foretinib 7.90 µmol/kg | 6 | 695.24 + 63.91 | 40.76% | 5.6% |
| Compound D1 7.90 µmol/kg | 6 | 1082.48 ± 52.47 | -0.21% | -1.8% |
| Control compound C1 7.90 µmol/kg | 6 | 727.22 ± 35.59 | 37.37% | 8.8% |
| Control compound C1 39.5 µmol/kg | 6 | 634.32 ± 22.93 | 46.92% | 2.2% |
| Control compound C1 79 µmol/kg | 6 | 390.71 ± 49.71 | 71.99% | 1.6% |
| Compound S1 7.90 µmol/kg | 6 | 282.05 ± 21.73 | 83.08% | 1.6% |
| Compound S1 39.5 µmol/kg | 6 | 148.59 ± 19.95 | 96.82% | 5.7% |
| Compound S1 79 µmol/kg | 6 | 62.59 ± 9.01 | 105.85% | 0.3% |
| Compound S2 7.90 µmol/kg | 6 | 310.25 ± 20.45 | 80.38% | 3.2% |
| Compound S2 39.5 µmol/kg | 6 | 185.59 ± 15.95 | 93.23% | 5.9% |
| Compound S2 79 µmol/kg | 6 | 105.59 ± 12.01 | 101.48% | 0.8% |
| Compound S12 7.90 µmol/kg | 6 | 508.42 ± 45.59 | 59.95% | 3.1% |
| Compound S12 39.5 µmol/kg | 6 | 413.12 ± 22.83 | 69.78% | 3.5% |
| Compound S12 79 µmol/kg | 6 | 285.51 ± 39.71 | 82.93% | 2.8% |
| Compound S15 7.90 µmol/kg | 6 | 727.22 ± 35.59 | 37.39% | -1.5% |
| Compound S15 39.5 µmol/kg | 6 | 634.32 ± 22.93 | 46.97% | 2.5% |
| Compound S15 79 µmol/kg | 6 | 390.71 ± 49.71 | 72.09% | 2.3% |

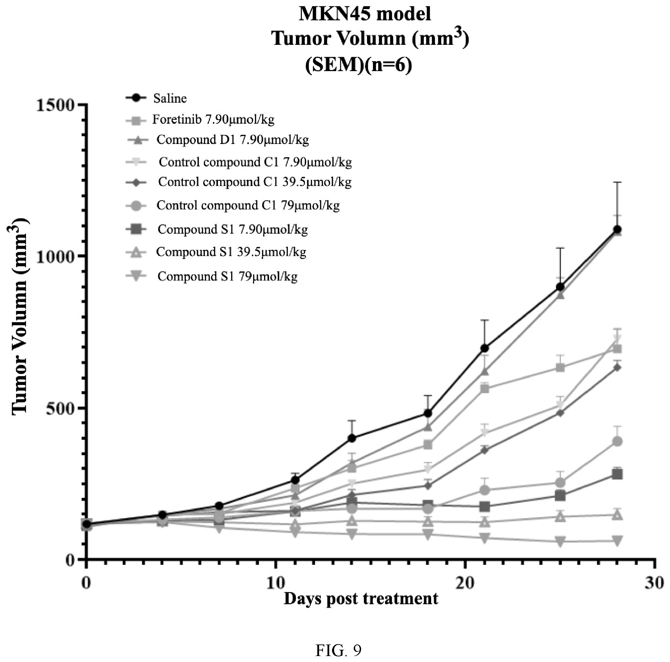

As shown in FIG. 9 and Table 5, which show an effect of test substances on growth of mouse xenograft MKN45. In the MKN45 xenograft model, compared with the normal saline group, foretinib, the control compound C1, the compound S1, the compound S2, the compound S12, and the compound S15 all exhibited good tumor inhibition effects and showed a dose-dependent effect, while the compound D1 did not exhibit a tumor inhibition effect. Compared with the foretinib positive control group, at an equimolar dose, the control compound C1, the compound S1, the compound S2, and the compound S12 all exhibited certain efficacy advantages, and effects of the compound S1, the compound S2, the compound S12, and the compound S15 were all better than that of the compound C1.

### Embodiment 13: Efficacy study of compound S1, compound S9, and compound S10 in treatment of HUH7 tumor model

### I. Experimental materials

Test substances: control compound C1, a low dose of 3.95 µmol/kg, a medium dose of 7.90 µmol/kg, and a high dose of 23.71 µmol/kg; compound S1, a low dose of 3.95 µmol/kg, a medium dose of 7.90 µmol/kg, and a high dose of 23.71 µmol/kg; compound S9, a low dose of 3.95 µmol/kg, a medium dose of 7.90 µmol/kg, and a high dose of 23.71 µmol/kg; and compound S10, a low dose of 3.95 µmol/kg, a medium dose of 7.90 µmol/kg, and a high dose of 23.71 µmol/kg.

Test animals: Balb/c Nude mice, 7 to 8 weeks of age, female, obtained from Shanghai Lingchang Biotechnology Co., Ltd.

### II. Preparation of tumor model:

1) Cell culture: HUH7 cells were obtained from Nanjing KeBai Biotech Co., Ltd. The cells were cultured in DMEM medium containing 10 % fetal bovine serum in a 37 °C, 5 % CO₂ incubator. Passage was performed once every three days, and cells within 15 passages were used.
2) Cell inoculation: A concentration of cells was adjusted to 5.0 × 10⁷/mL with DMEM basic medium. The cells were inoculated subcutaneously into a right back of the mice, 0.1 mL per mouse.
3) Treatment: When a tumor volume reached 100 mm³ to 200 mm³, the mice were randomly divided into 9 groups and treatment was started. 6 mice were included in each group, which were respectively a normal saline negative control group, a foretinib positive control group, a compound D1 control group, a control compound C1 low dose group, a control compound C1 medium dose group, a control compound C1 high dose group, a compound S1 low dose group, a compound S1 medium dose group, and a compound S1 high dose group. A day of grouping was set as D0, and experimental results were D36 (i.e., results of the 36th day).

Administration results are shown in Table 6.

**Table 6: Effects of corresponding compounds and control groups on tumor inhibition**

| Group | N | Tumor size (mean + SEM) (mm³) | Tumor growth inhibition rate TGI (%) | Body weight vs D0 (%) |
|---|---|---|---|---|
| | | D36 | D36 | D36 |
| Normal saline | 6 | 2684.01 ± 899.96 | - | 12.8% |
| Foretinib | 6 | 2509.13 ± 902.2 | 6.83% | 18.4% |
| Compound D1 | 6 | 1614.49 ± 323.28 | 41.77% | 8.8% |
| Control compound C1 3.95 µmol/kg | 6 | 1608.75 ± 527.38 | 41.96% | 8.2% |
| Control compound C1 7.90 µmol/kg | 6 | 1448.66 ± 254.7 | 48.36% | 11.6% |
| Control compound C1 23.71 µmol/kg | 6 | 1102.82 ± 138.2 | 61.88% | 9.9% |
| Compound S 1 3.95 µmol/kg | 6 | 1470.04 ± 355.61 | 47.49% | 7.0% |
| Compound S1 7.90 µmol/kg | 6 | 1271.77 ± 258.25 | 55.19% | 12.8% |
| Compound S1 23.71 µmol/kg | 6 | 685.83 ± 79.86 | 78.09% | 18.4% |
| Compound S9 3.95 µmol/kg | 6 | 1523.14 ± 331.31 | 45.28% | 10.1% |
| Compound S9 7.90 µmol/kg | 6 | 1391.78 ± 289.35 | 50.39% | 13.7% |
| Compound S9 23.71 µmol/kg | 6 | 715.64 ± 83.76 | 76.77% | 15.1% |
| Compound S10 3.95 µmol/kg | 6 | 1680.04 ± 215.61 | 39.16% | 11.3% |
| Compound S10 7.90 µmol/kg | 6 | 1470.73 ± 273.25 | 47.32% | 15.3% |
| Compound S10 23.71 µmol/kg | 6 | 745.81 ± 89.76 | 75.59% | 17.2% |

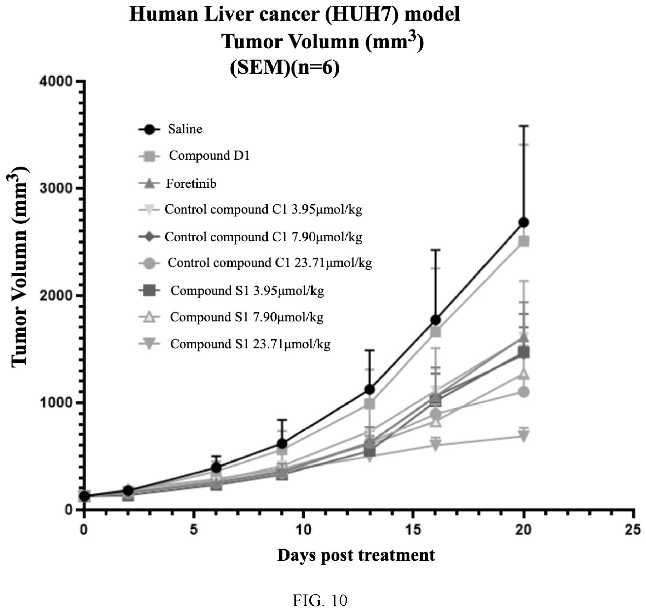

As shown in FIG. 10 and Table 6, in the HUH7 xenograft model, compared with the normal saline group, foretinib, the control compound C1, the compound S1, the compound S9, and the compound S10 all exhibited good tumor inhibition effects and showed a dose-dependent effect, while the compound D1 did not exhibit a tumor inhibition effect. Compared with the foretinib positive control group, at an equimolar dose, the control compound C1, the compound S1, the compound S9, and the compound S10 all exhibited certain efficacy advantages, and effects of the compound S1 and the compound S9 were both better than that of the compound C1.

### Embodiment 14: Efficacy study of compound S19 and compound S21 in treatment of MDA-MB-231 tumor model

### I. Experimental materials

Test substances: control compound C1, a low dose of 3.95 µmol/kg, a medium dose of 7.90 µmol/kg, and a high dose of 23.71 µmol/kg; S21, a low dose of 3.95 µmol/kg, a medium dose of 7.90 µmol/kg, and a high dose of 23.71 µmol/kg; and S19, a low dose of 3.95 µmol/kg, a medium dose of 7.90 µmol/kg, and a high dose of 23.71 µmol/kg.

Test animals: BALB/c Nude mice, 7 to 8 weeks of age, female, obtained from Shanghai Lingchang Biotechnology Co., Ltd.

### II. Preparation of tumor model:

1) Cell culture: MDA-MB-231 cells were obtained from the Cell Bank of the Chinese Academy of Sciences. The cells were cultured in RPMI-1640 medium containing 10 % fetal bovine serum in a 37 °C, 5 % CO₂ incubator. Passage was performed once every three days, and cells within 15 passages were used.
2) Cell inoculation: A concentration of cells was adjusted to 2.0 × 107/mL with RPMI-1640 basic medium. The cells were inoculated subcutaneously into a right back of the mice, 0.1 mL per mouse.
3) Treatment: When a tumor volume reached 80 mm³ to 200 mm³, the mice were randomly divided into 3 groups and treatment was started. 6 mice were included in each group, which were respectively a normal saline negative control group, a Niraparib group, and a Rucaparib group. A day of grouping was set as D0. Experimental results were D36 (i.e., results of the 36th day).

Administration results are shown in Table 7.

**Table 7: Effects of corresponding compounds and control groups on tumor inhibition**

| Group | N | Tumor size (mean ± SEM) (mm³) | Tumor growth inhibition rate TGI (%) | Body weight vs D0 (%) |
|---|---|---|---|---|
| | | D36 | D36 | D36 |
| Normal saline | 6 | 1579.34 + 134.47 | - | -3.0 % |
| Foretinib | 6 | 883.21 ± 63.91 | 47.70 % | 6.7 % |
| Compound D1 | 6 | 1382.48 ± 52.47 | 0.13 % | 2.7 % |
| Control compound C1 3.95 µmol/kg | 6 | 931.25 ± 35.59 | 44.40 % | 6.8 % |
| Control compound C1 7.90 µmol/kg | 6 | 834.32 ± 22.93 | 51.05 % | 3.2 % |
| Control compound C1 23.71 µmol/kg | 6 | 590.41 ± 49.71 | 67.77 % | 1.8 % |
| Compound S19 3.95 µmol/kg | 6 | 682.35 ± 20.45 | 61.46 % | 4.2 % |
| Compound S19 7.90 µmol/kg | 6 | 557.34 ± 15.95 | 70.03 % | 5.5 % |
| Compound S19 23.71 µmol/kg | 6 | 462.59 ± 12.01 | 76.52 % | 3.8 % |
| Compound S21 3.95 µmol/kg | 6 | 610.75 ± 45.59 | 66.37 % | 4.1 % |
| Compound S21 7.90 µmol/kg | 6 | 585.12 ± 22.83 | 68.13 % | 5.5 % |
| Compound S21 23.71 µmol/kg | 6 | 425.03 ± 39.71 | 79.10 % | 3.8 % |

As shown in Table 7, in the MDA-MB-231 tumor model, compared with the normal saline group, foretinib, the control compound C1, the compound S19, and the compound S21 all exhibited good tumor inhibition effects and showed a dose-dependent effect, while the compound D1 did not exhibit a tumor inhibition effect. Compared with the foretinib positive control group, at an equimolar dose, the control compound C1, the compound S19, and the compound S21 all exhibited certain efficacy advantages.

### Embodiment 15: Toxicity determination of foretinib, compound D1, Niraparib group, Rucaparib group, Palbociclib, S1, S18, S19, and S20

Experimental purpose: To understand the acute toxicity of a drug organism of the present invention by determining an intravenous MTD experiment in mice.

Test substances: Foretinib, compound D1, Niraparib, Rucaparib, Palbociclib, S1, S18, S19, and S20 were dissolved with DMSO, and then diluted to a corresponding dose with normal saline during the experiment.

Animals: Grade I BALB/C mice (obtained from Shanghai Lingchang Biotechnology Co., Ltd.), weighing 19 to 21 g, all female.

Methods and results: 84 female BALB/C mice to be tested, weighing 19 to 21 g, were randomly divided into 14 groups according to body weight, with 6 mice in each group. According to doses shown in Table 8 below, a normal saline group, foretinib, the compound D1, a Niraparib group, a Rucaparib group, a Palbociclib group, S1, S18, S19, and S20 were respectively injected intravenously at one time. Each of S1, S18, S19, and S20 had a high-dose group. An administration volume for each mouse was 0.2 mL. Observation was performed continuously for 17 days. Whether the animals showed piloerection, ruffled and lackluster fur, lethargy, hunched posture, hypersensitivity reaction, etc. was observed daily. Body weight and death were recorded. Blood samples were collected for a complete blood count on the 3rd, 5th, and 14th days. The animals were dissected on the 14th day, and the heart, liver, kidney, lung, spleen, and pancreas were collected for HE staining observation.

**Table 8: Comparison of mortality results of test mice receiving different doses of compound injections and normal saline respectively**

| Group | | Dose (µmol/kg) | Animals (units) | Deaths (units) |
|---|---|---|---|---|
| 1 | Normal saline | 0 | 6 | 0 |
| 2 | Foretinib | 50 | 6 | 4 |
| 3 | Compound D1 | 50 | 6 | 0 |
| 4 | Niraparib | 50 | 6 | 4 |
| 5 | Rucaparib | 50 | 6 | 5 |
| 6 | Palbociclib | 50 | 6 | 6 |
| 7 | S1 | 50 | 6 | 0 |
| 8 | S1 | 200 | 6 | 0 |
| 9 | S18 | 50 | 6 | 0 |
| 10 | S18 | 200 | 6 | 0 |
| 11 | S19 | 50 | 6 | 0 |
| 12 | S19 | 200 | 6 | 0 |
| 13 | S20 | 50 | 6 | 0 |
| 14 | S20 | 200 | 6 | 0 |

In summary, it can be seen that in the same dose group of 50 µmol/kg, compared with the foretinib group, the Niraparib group, the Rucaparib group, and the Palbociclib group, the animals in the groups of the compounds S1, S18, S19, and S20 provided by the present invention did not show piloerection, ruffled and lackluster fur, lethargy, hunched posture, hypersensitivity reaction, or death. Moreover, in the high-dose group of 200 µmol/kg, no death occurred. This further indicates that the toxicity of an albumin-coupled drug is significantly lower than that of a non-coupled drug, and the therapeutic window index is greatly improved.

### Embodiment 16: Efficacy study of compound S1 in treatment of colorectal cancer CT-26 tumor model

Animals: 36 female Balb/c mice.

Experimental method: Mouse colorectal cancer CT-26 cells were inoculated subcutaneously to construct a mouse xenograft model. When the xenograft model reached a volume of 100 to 200 mm³, the mice were randomly divided into 6 groups, including:
a negative control group, to which normal saline was administered;
a positive control group, to which 5 mg/kg (7.90 µmol/kg) of foretinib was administered;
an S1 low-dose group, to which 15.7 mg/kg (7.90 µmol/kg) of the compound S1 was administered;
an S1 medium-dose group, to which 47 mg/kg (12.71 µmol/kg) of the compound S1 was administered;
an S1 medium-high-dose group, to which 70.4 mg/kg (33.56 µmol/kg) of the compound S1 was administered; and
an S1 high-dose group, to which 93.9 mg/kg (47.42 µmol/kg) of the compound S1 was administered.

A mode of administration was intravenous administration, with an administration volume of 10 mL/kg for each. Dosing was performed twice a week for four consecutive weeks.

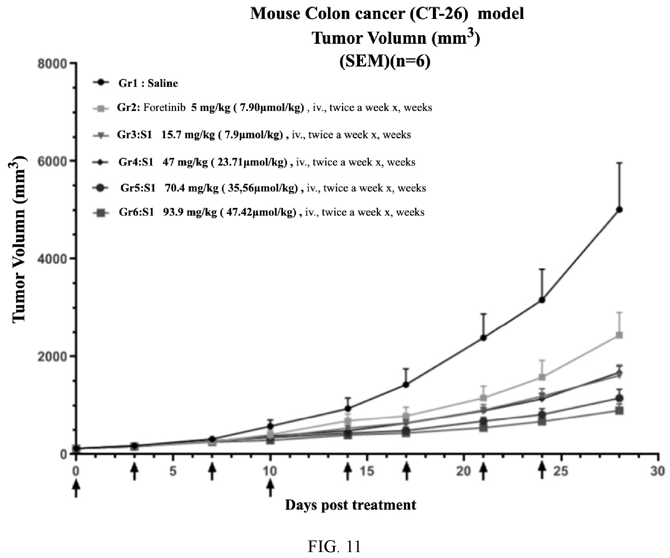

Experimental results: Administration results are shown in FIG. 11, which shows the tumor volume after the mouse colorectal cancer CT-26 model was given different doses of S1. Compared with the normal saline group, the compound S1 exhibited a good tumor inhibition effect and showed a dose-dependent effect. At an equimolar dose, the compound S1 exhibited a certain efficacy advantage, and an effect of the compound S1 was better than that of foretinib.

### Embodiment 17: Efficacy study of compound S1 in combination with anti-PD-1 antibody in treatment of colorectal cancer CT-26 tumor model

Animals: 24 female Balb/c mice.

Test method: Mouse colorectal cancer CT-26 cells were inoculated subcutaneously to construct a mouse xenograft model. When the xenograft model reached a volume of 100 to 200 mm³, the mice were randomly divided into 4 groups, including:
a negative control group, to which normal saline was administered;
an S1 monotherapy dose group, to which 7.7 mg/kg (3.95 µmol/kg) of the compound S1 was administered;
an anti-PD-1 antibody treatment group, to which 5 mg/kg of the Anti-mPD-1 antibody was administered; and
a combination treatment group, to which 7.7 mg/kg (3.95 µmol/kg) of the compound S1 was administered in combination with 5 mg/kg of the Anti-mPD-1 antibody.

A mode of administration was intravenous administration, with an administration volume of 10 mL/kg for each. Dosing was performed twice a week for three consecutive weeks.

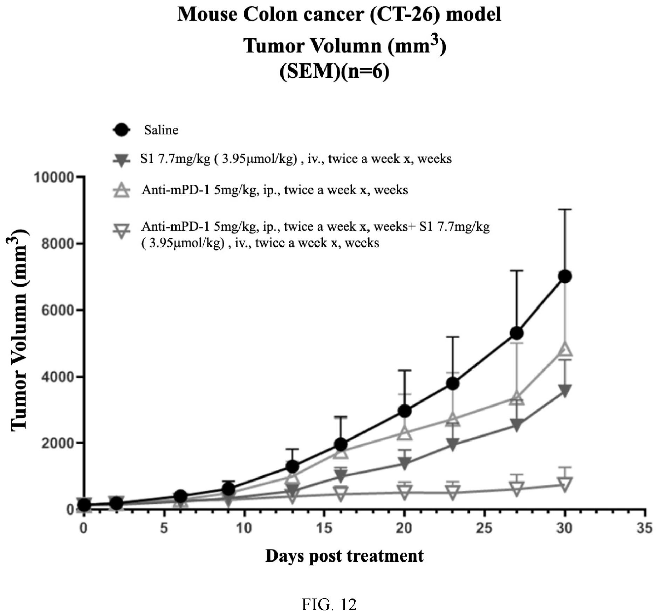

Test results: Administration results are shown in FIG. 12, which shows the tumor volume after the mouse colorectal cancer CT-26 model was given different modes of administration. Compared with the S1 monotherapy dose group and the anti-PD-1 treatment group, the combination treatment group exhibited a better tumor inhibition effect, indicating that the combination of the compound S1 and the anti-PD-1 antibody has a synergistic effect, and the compound S1 combined with the anti-PD-1 antibody can cure tumor at a low dose.

### Embodiment 18: Efficacy study of compound S1 in treatment of human gastric cancer MKN45 tumor model

Animals: 36 female nude mice.

Test method: Human gastric cancer MKN45 cells were inoculated subcutaneously to construct a nude mouse xenograft model. When the xenograft model reached a volume of 100 to 200 mm³, the mice were randomly divided into 6 groups, including:
a negative control group, to which normal saline was administered;
a positive control group, to which 5 mg/kg (7.90 µmol/kg) of the compound D1 was administered;
an S1 low-dose group, to which 15.8 mg/kg (7.90 µmol/kg) of the compound S1 was administered;
an S1 medium-dose group, to which 79.2 mg/kg (39.5 µmol/kg) of the compound S1 was administered;
an S1 medium-high-dose group, to which 158.3 mg/kg (79 µmol/kg) of the compound S1 was administered; and
an S1 high-dose group, to which 200 mg/kg (99.8 µmol/kg) of the compound S1 was administered.

A mode of administration was intravenous administration, with an administration volume of 10 mL/kg for each. Dosing was performed twice a week for three consecutive weeks.

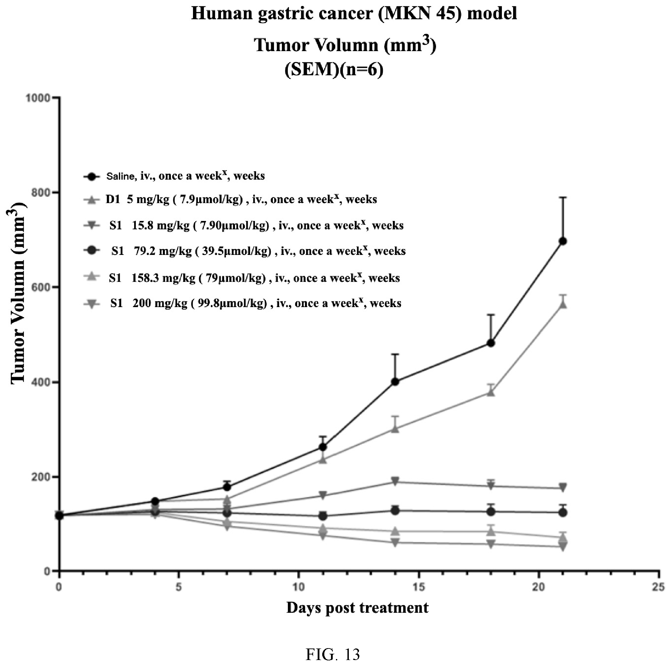

Test results: Administration results are shown in FIG. 13, which shows the tumor volume after the human gastric cancer MKN45 model was given different doses of S1. Compared with the normal saline group, the compound S1 exhibited a good tumor inhibition effect and showed a dose-dependent effect. At an equimolar dose, the compound S1 exhibited a certain efficacy advantage, and an effect of the compound S1 was better than that of the compound D1.

### Embodiment 19: Beagle dog toxicity test of compound S1

Animals: 40 Beagle dogs were selected and randomly divided into 4 groups, with 10 dogs in each group, half male and half female.

Test method: The four groups were respectively given normal saline, and 9, 18, and 36 mg/kg of the compound S1. Dosing was performed once a week for 4 consecutive weeks. Subsequently, drug withdrawal was followed by a recovery period after drug withdrawal for 4 weeks. In the test, the following examinations were performed: general observation, body weight, food consumption, clinical pathology (including hematology, blood biochemistry, and coagulation), body temperature, electrocardiogram, ophthalmic examination, urine, gross anatomy, organ weight, bone marrow smear, histopathological examination, and toxicokinetics.

Experimental results indicated that animals in each dose group showed good tolerance, and the HNSTD dose was 36 mg/kg.

It should be noted that an "effective amount" in the present invention refers to an amount of a component sufficient to produce a desired response. A specific effective amount depends on various factors, such as a specific condition to be treated, physical conditions of a patient (such as body weight, age, or gender of the patient), duration of treatment, a therapy administered concurrently (if any), and a specific formulation used. The "effective amount" also refers to an amount at which toxicity or negative effects of a kinase inhibitor of the present application are not as much as positive curative effects brought thereby.

In the present disclosure, a therapeutically effective amount of the compound of formula (I) or a pharmaceutically acceptable salt thereof may be equivalent to a therapeutically effective amount of a conventional drug molecule D. In some cases, since a linker of the present disclosure (i.e., the A group) is coupled, higher activation efficiency and anti-tumor effects are achieved. Therefore, a therapeutically effective amount of the compound of formula (I) of the present disclosure or a pharmaceutically acceptable salt thereof is lower than a therapeutically effective amount of the conventional drug molecule D. In summary, the aforementioned embodiments are only preferred embodiments of the present invention and are not used to limit the protection scope of the present invention. Any modifications, equivalent replacements, improvements, etc. made within the spirit and principle of the present invention shall be included in the protection scope of the present invention.

## Claims

1. A kinase inhibitor activated in a tumor microenvironment, comprising a compound having a structure shown as the following formula (I), a stereoisomer thereof, or a pharmaceutically acceptable salt thereof:
R-A-C-D (I)
wherein,
R is a modifying group for improving physicochemical properties of a drug;
A is a protease-cleavable amino acid linker;
C is a steric hindrance group or a self-immolative group; and
D is a drug molecule.

2. The kinase inhibitor activated in a tumor microenvironment according to claim 1, wherein the R is a modifying group with an MI structure shown as a structure of the following formula (II): 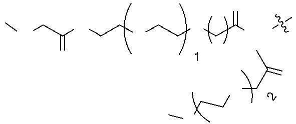 or 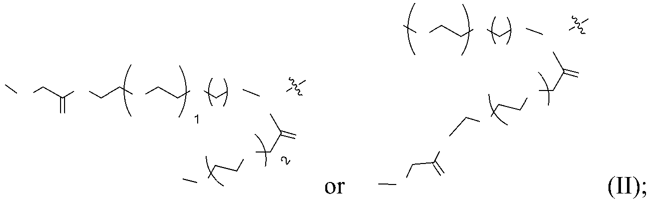 wherein,
MI is a structure having a conjugated system and an addition acceptor;
Rc is a structure for increasing water solubility, compound rigidity, or expanding spatial distance;
n₁ is a positive integer from 1 to 12, n₂ is a positive integer from 1 to 12, and n₃ is 1 or 2;
X₁ is a trifunctional group; and
a wavy line represents a connection position of the R and the A.

3. The kinase inhibitor activated in a tumor microenvironment according to claim 2, wherein the MI is selected from: 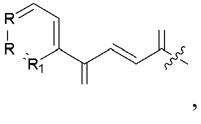 wherein R₁, R₂, and R₃ are CH or N;
or 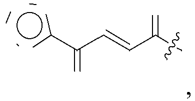 wherein a cyclic structure in the structure is a five-membered ring including any one of N, O, or S, and R₄, R₅, R₆, and R₇ are one of CH, N, O, or S;
or 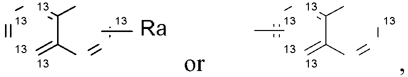
wherein R₈, R₉, R₁₀, R₁₁, R₁₂, R₁₃, R₁₄, and R₁₅ are C, CH, or N, and Ra and Rb are 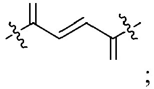
or 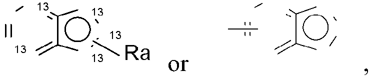 wherein a cyclic structure in the structure is a five-membered ring including any one of N, O, or S, and R₁₆, R₁₇, and R₁₈ are one of C, CH, N, O, or S, R₁₉, R₂₀, R₂₁, and R₂₂ are C, CH, or N, and Re and Rf are

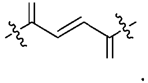

4. The kinase inhibitor activated in a tumor microenvironment according to claim 2, wherein the Rc is selected from C₁-₁₂ alkyl, C₁₋₁₂ oxyalkyl-C₁₋₁₂ alkyl, C₁₋₁₂ alkyl-C₃₋₈ cycloalkyl, (C₁₋₄ alkyl-O)p-C₁₋₁₂ alkyl, phenyl-C₁₋₁₂ alkyl, C₃₋₈ cycloalkyl, C₁₋₁₂ alkyl-C₃₋₈ cycloalkyl-C₁₋₁₂ alkyl, C₁₋₁₂ alkyl-phenyl-C₁₋₁₂ alkyl, or C₁₋₁₂ alkylcarbonylamino-(C₁₋₄ alkyl-O)q-C₁₋₁₂ alkyl;
wherein, p is a positive integer from 1 to 12, q is a positive integer from 1 to 12, the phenyl is one or two substituted phenyls, and a substituent in the substituted phenyl is a halogen atom.

5. The kinase inhibitor activated in a tumor microenvironment according to claim 2, wherein the X₁ is selected from lysine, Lys, or glutamic acid, Glu;
when the X₁ is the lysine, a structure of the X₁ is 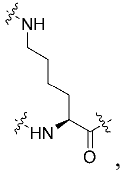
wherein a carboxyl terminus of the X₁ is connected to the A, and two amino termini of the X₁ are connected in a one-to-one correspondence to a side chain containing n1 or a side chain containing n2;
when the X₁ is the glutamic acid, Glu, a structure of the X₁ is 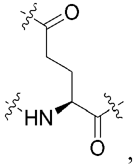
wherein an amino terminus is connected to the side chain containing n1 or the side chain containing n2; a carboxyl terminus adjacent to an amino group on the glutamic acid is connected to the A, and a carboxyl terminus remote from the amino group on the glutamic acid is connected to the side chain containing n1 or the side chain containing n2.

6. The kinase inhibitor activated in a tumor microenvironment according to claim 1, wherein the A is -Ala-Ala-Asn-.

7. The kinase inhibitor activated in a tumor microenvironment according to claim 1, wherein the C is 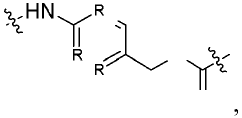 wherein R₂₃, R₂₄, and R₂₅ are CH or N.

8. The kinase inhibitor activated in a tumor microenvironment according to claim 1, wherein the drug molecule D is one of the following chemical formulas:

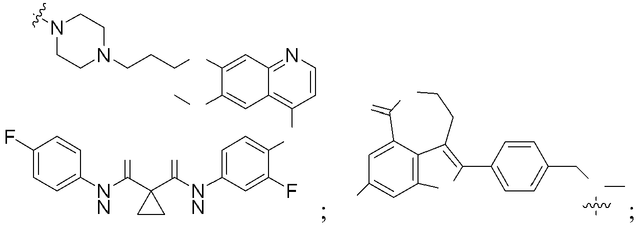

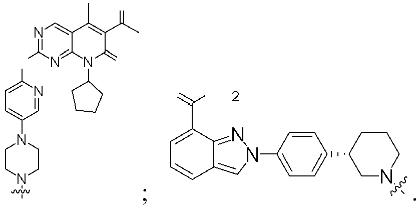

9. The kinase inhibitor activated in a tumor microenvironment according to claim 1, wherein a structure of the kinase inhibitor is selected from:
| Name | Compound structure |
|---|---|
| Compound S1 | 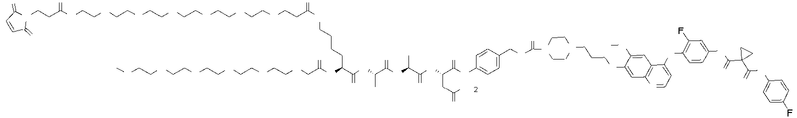 |
| Compound S2 | 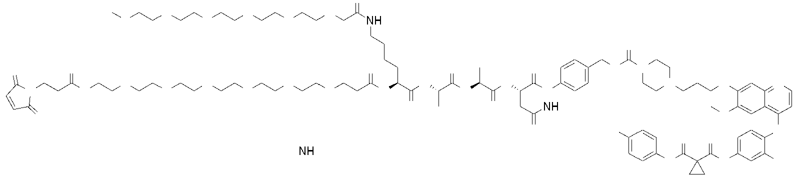 |
| Compound S3 | 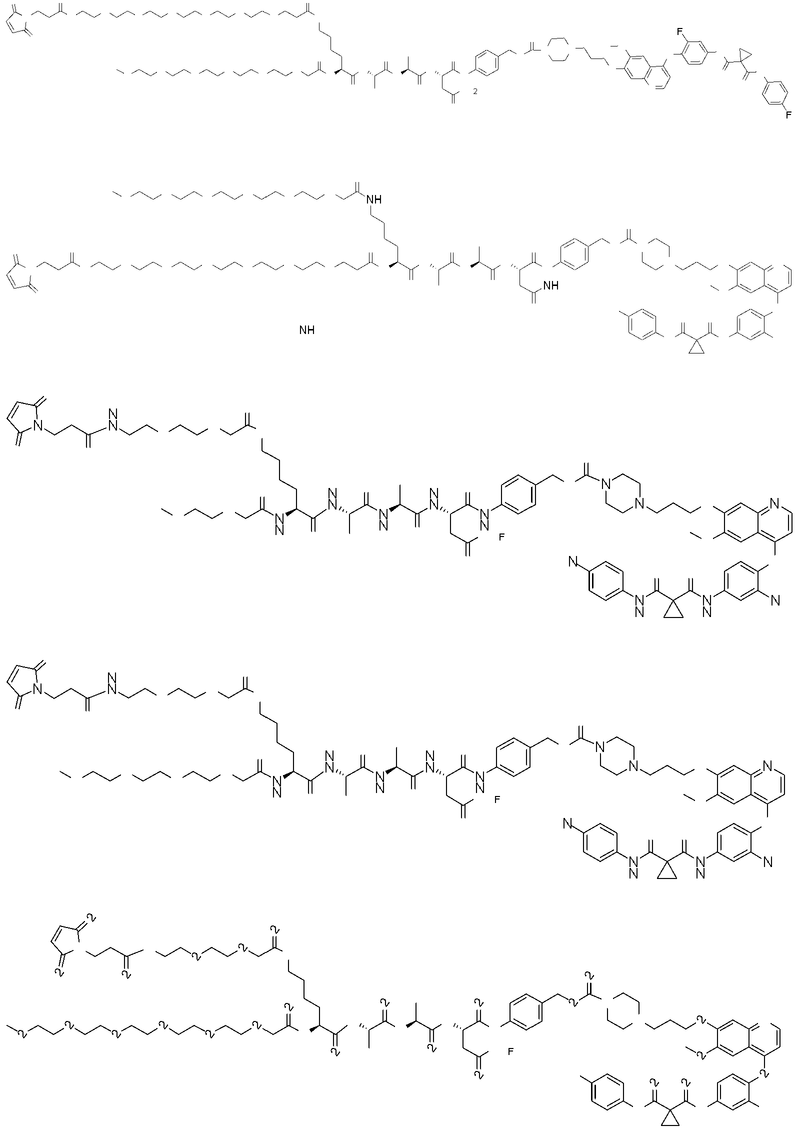 |
| Compound S4 | 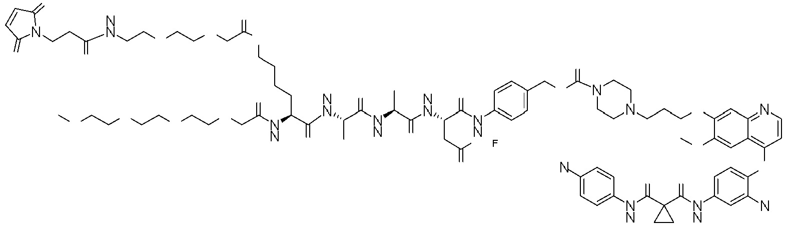 |
| Compound S5 | 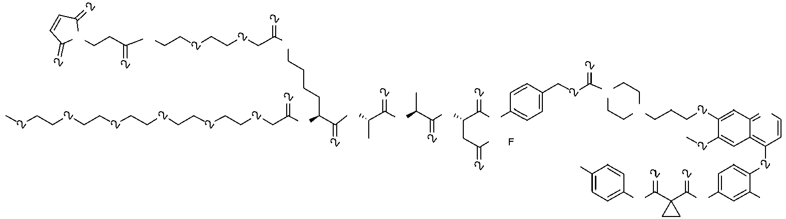 |
| Compound S6 | 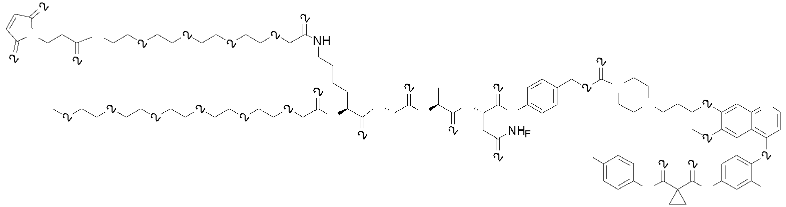 |
| Compound S7 | 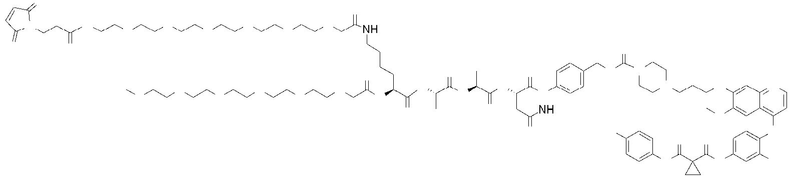 |
| Compound S8 | 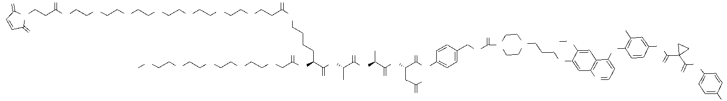 |
| Compound S9 | 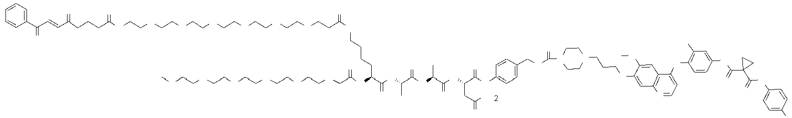 |
| Compound S10 | 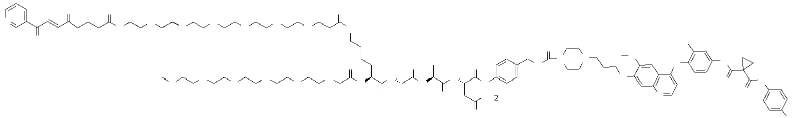 |
| Compound S11 | 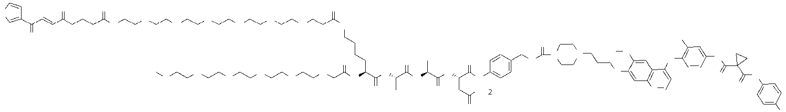 |
| Compound S12 | 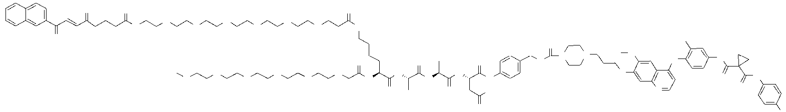 |
| Compound S13 | 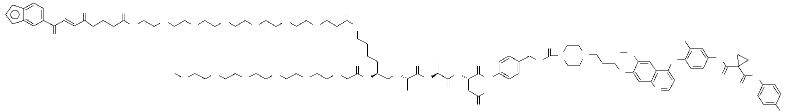 |
| Compound S14 | 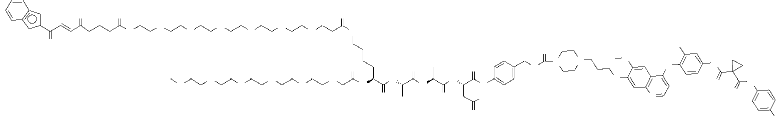 |
| Compound S15 | 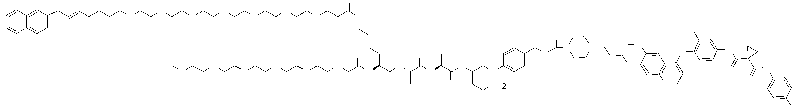 |
| Compound S16 | 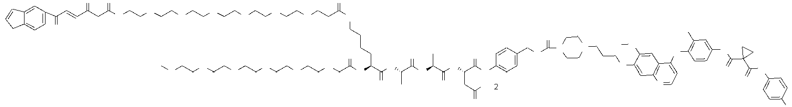 |
| Compound S17 | 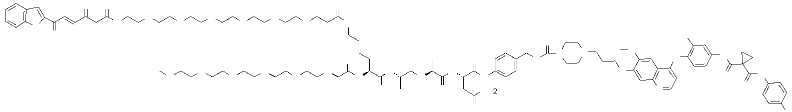 |
| Compound S18 | 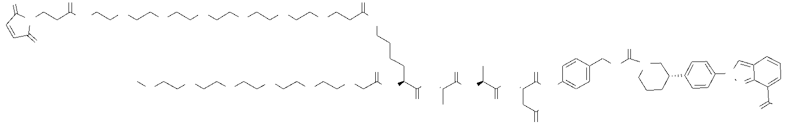 |
| Compound S19 | 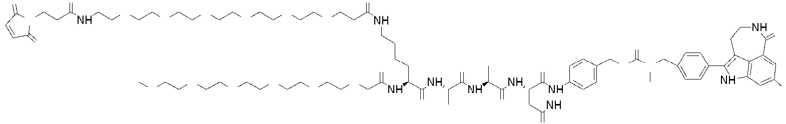 |
| Compound S20 | 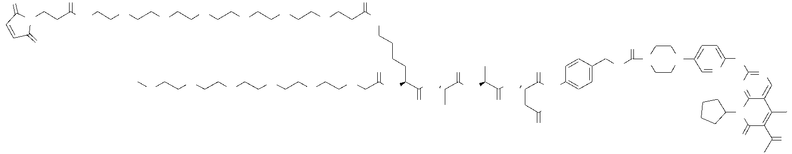 |
| Compound S21 | 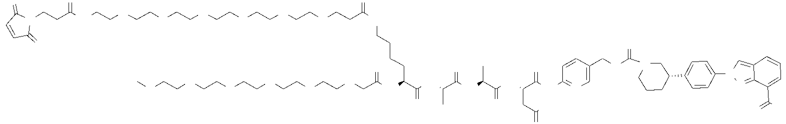 |
| Compound S22 | 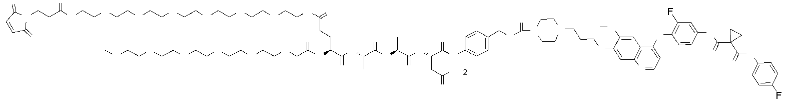 |
| Compound S23 | 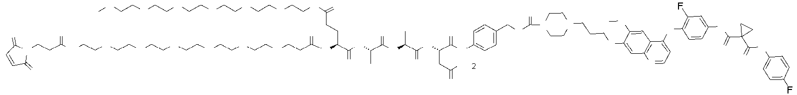 |

10. A pharmaceutical composition, comprising the kinase inhibitor activated in a tumor microenvironment according to any one of claims 1 to 9, a pharmaceutically acceptable carrier thereof, or a pharmaceutical composition.

11. A use of the kinase inhibitor activated in a tumor microenvironment according to any one of claims 1 to 9 in the preparation of a medicament for treating cancer, the cancer comprising colorectal cancer, ovarian cancer, fallopian tube cancer, peritoneal cancer, breast cancer, gastric cancer, liver cancer, head and neck cancer, prostate cancer, and pancreatic cancer.

12. A use of the kinase inhibitor activated in a tumor microenvironment according to any one of claims 1 to 9 in a medicament for treating cancer, comprising a use of the kinase inhibitor activated in a tumor microenvironment according to any one of claims 1 to 9 or a pharmaceutically acceptable salt thereof, or the pharmaceutical composition according to claim 10 or a pharmaceutically acceptable salt thereof, in combination with an anti-PD-1 antibody in the preparation of a medicament for combined treatment of a tumor.

13. A method for treating or preventing cancer, or inhibiting cancer cell proliferation, comprising: administering the kinase inhibitor activated in a tumor microenvironment according to any one of claims 1 to 9 to a subject.

14. The method according to claim 13, further comprising: administering an immune checkpoint inhibitor to the subject in combination with the kinase inhibitor activated in a tumor microenvironment according to any one of claims 1 to 9.

15. The method according to claim 14, wherein the immune checkpoint inhibitor is an anti-PD-1 antibody drug.
